# NEW EUROPEAN PATENT SPECIFICATION

(11) **EP 1 180 020 B2**
(45) Date of publication and mention of the opposition decision: **24.06.2009**
(45) Mention of the grant of the patent: 14.12.2005
(21) Application number: 00939365.3
(22) Date of filing: 25.05.2000
(51) Int. Cl.: A61K 9/16

(54) **POROUS DRUG MATRICES AND METHODS OF MANUFACTURE THEREOF**
PORÖSE ARZNEISTOFFMATRIZEN UND DEREN HERSTELLUNGSVERFAHREN
MATRICES MEDICAMENTEUSES POREUSES ET PROCEDES DE FABRICATION ASSOCIES

(30) Priority: 27.05.1999 US 136323 P; 08.10.1999 US 158659 P; 04.11.1999 US 433486; 02.03.2000 US 186310 P
(43) Date of publication of application: 20.02.2002
(60) Divisional application: 05027194.9
(73) Proprietor: Acusphere, Inc., Watertown, MA 02472 (US)
(72) Inventor: STRAUB, Julie, Winchester, MA 01890 (US); BERNSTEIN, Howard, Cambridge, MA 02138 (US); CHICKERING, Donald, E. III, Framingham, MA 01701 (US); KHATAK, Sarwat, Hadley-Massachusetts 01035 (US); RANDALL, Greg, Stoneham, MA 02180 (US)
(74) Representative: HOFFMANN EITLE
(86) International application number: PCT/US2000/014578
(87) International publication number: WO 2000/072827

(56) References cited:
- EP-A- 0 655 237
- WO-A-98/31346
- WO-A-99/56731
- WO-A1-98/31346
- WO-A1-99/56731
- DE-A- 3 713 326
- GB-A- 1 265 615
- US-A- 3 948 245
- US-A- 4 687 660

## Description

### Background of the Invention

This invention generally relates to formulations of drugs, especially drugs having low solubility, and more particularly to methods of making formulations of such drugs to enhance their rate of dissolution.

The bioavailability of a drug can be limited by poor dissolution of the drug into aqueous bodily fluids following administration. This rate-limiting step may therefore be critical to rapidly attaining therapeutically effective drug levels.

Traditional approaches to parenteral delivery of poorly soluble drugs include using large volumes of aqueous diluents, solubilizing agents, detergents, non-aqueous solvents, or non-physiological pH solutions. These formulations, however, can increase the systemic toxicity of the drug composition or damage body tissues at the site of administration.

For example, paclitaxel is a natural product which has been shown to possess cytotoxic and antitumor activity. While having an unambiguous reputation of tremendous therapeutic potential, paclitaxel as a therapeutic agent has some patient related drawbacks. These stem, in part, from its extremely low solubility in water, which makes it difficult to provide in suitable dosage form. Because of paclitaxel's poor aqueous solubility, the current approved (U.S. FDA) clinical formulation consists of a 6 mg/ml solution of paclitaxel in 50% polyoxyethylated castor oil (CREMOPHOR EL™) and 50% dehydrated alcohol. Am. J. Hosp. Pharm., 48:1520-24 (1991). In some instances, severe reactions, including hypersensitivity, occur in conjunction with the CREMOPHOR™ administered in conjunction with paclitaxel to compensate for its low water solubility. As a result of the incidence of hypersensitivity reactions to the commercial paclitaxel formulations and the potential for paclitaxel precipitation in the blood, the formulation must be infused over several hours. In addition, patients must be pretreated with steroids and antihistamines prior to the infusion. In response to the hypersensitivity related to the CREMOPHOR™, the increasing recognition of paclitaxel's promise as an antineoplastic, and the undesirability of having to infuse the paclitaxel over several hours, there remains a need to develop improved formulations of the paclitaxel which can be administered as bolus injections. Similarly, it would be advantageous to administer docetaxel by bolus injection, rather than by infusion following dissolution of the drug in 100% Polysorbate 80, as is the current practice.

Other approaches to parenteral delivery of poorly soluble drugs have focused on the physical form of the drug itself. Since the dissolution rate of a drug particle is directly related to its surface area available to contact the aqueous media at the site of administration or site of absorption, methods of preparing drugs in nanoparticulate form have been developed in an effort to maximize the drug surface area, as described, for example, in U.S. Patent No. 5,534,270 to De Castro and U.S. Patent No. 5,587,143 to Wong. Nanoparticles, however, can be difficult to produce and maintain in a stable form due to the tendency of the nanoparticles to flocculate or agglomerate, particularly without the presence of surface modifying agents adsorbed or coated onto the particles. Furthermore, milling or wet grinding techniques, which are typically employed for nanonization, can be undesirable, as it can take several days to process a single batch, scaling-up of the milling or grinding process can be difficult and/or costly, the process can be difficult to conduct aseptically, and it is difficult to eliminate shedding of milling media into the product.

Other efforts directed at enhancing the rate of dissolution have focused on delivering the drug as a dispersion in a water-soluble or biodegradable matrix, typically in the form of polymeric microparticles. For example, the dissolution rate of dexamethasone reportedly was improved by entrapping the drug in chitosan microspheres made by spray-drying (Genta, et al., S.T.P. Pharma Sciences 5(3):202-07 (1995)). Similarly, others have reported enhanced dissolution rates by mixing a poorly soluble drug powder with a water-soluble gelatin, which purportedly makes the surface of the drug hydrophilic (Imai, et al., J. Pharm. Pharmacol., 42:615-19 (1990)).

Related efforts have been directed to forming relatively large, porous matrices of low solubility drugs. For example, Roland & Paeratakul, "Spherical Agglomerates of Water-Insoluble Drugs," J. Pharma. Sci., 78(11):964-67 (1989) discloses preparing beads having a low solubility drug content up to 98%, wherein the beads have a porous internal structure. Such large beads, however, are unsuitable for pareriteral administration, and the beads have less surface area and slower dissolution rates than smaller particles. Document WO 99/56731, relevant pursuant to Article 54(3)(4) EPC, discloses matrices formed of polymer and hydrophobic compounds in order to alter drug release kinetics.

It is therefore an object of the present invention to provide compositions enhancing the dissolution rate of drugs, especially drugs having low aqueous solubility, and to provide methods of making such compositions.

It is another object of the present invention to provide compositions providing enhanced dissolution of drugs, especially drugs of low aqueous solubility, in a formulation suitable for administration by a variety of routes, including, but not limited to, parenteral, mucosal, oral, and topical administration, for local, regional, or systemic effect.

It is another object of the present invention to provide compositions providing enhanced dissolution of paclitaxel in a formulation suitable for administration by a variety of routes, including, but not limited to, parenteral, mucosal, oral, and topical administration, for local, regional, or systemic effect.

It is still another object of the present invention to provide compositions of paclitaxel or docetaxel without the required solubilizing agents present in current commercial formulations.

It is a further object of the present invention to provide compositions, particularly paclitaxel or docetaxel, for administration as a bolus injection instead of by infusion.

### Summary of the Invention

Drugs are provided in a porous matrix form wherein the dissolution rate of the drug is enhanced when the matrix is contacted with an aqueous medium. Pharmaceutical compositions of the present invention are specified in appended Claim 8. In a preferred embodiment, low aqueous solubility drugs are provided in a porous matrix form which forms microparticles when the matrix is contacted with an aqueous medium. The porous matrix with low aqueous solubility drugs yields upon contact with an aqueous medium microparticles having a mean diameter between about 0.1 and 5 µm and a total surface area greater than about 0.5 m²/mL. The dry porous matrix preferably is in a dry powder form having a TAP density less than or equal to 1.0 g/mL.

In a preferred embodiment, the drug is a taxane. For example, paclitaxel or docetaxel can be provided in a porous matrix form which forms nanoparticles and microparticles of the drug when the matrix is contacted with an aqueous medium.

The porous matrices that contain the drug are made using a method for making a porous matrix of drug comprising
(a) dissolving a drug in an organic volatile solvent to form a drug solution,
(b) combining at least one volatile solid pore forming agent with the drug solution to form an emulsion, suspension, or second solution containing the pore forming agent,
(c) incorporating at least one wetting agent into the emulsion, suspension or second solution, and
(d) removing the volatile solvent and pore forming agent from the emulsion, suspension, or second solution to yield the porous matrix of drug,
provided that the matrix is not formed by dissolving 18 grams of poly lactide-co-glycolide (50:50) (IV 0.4 dL/g Boehringer Ingelheim), 1.08 g of diarachidoylphosphatidylcholine in 600 mL of methylene chloride to form a polymer solution; dissolving 38.9 mg of Eosin Y in 38.9 mL of a 0.18 g/ml ammonium bicarbonate solution to form an eosin solution; emulsifying the eosin solution with the polymer solution using a homogenizer at 7000 rpm for 8 minutes; pumping the solution at a flowrate of 20 mL/min and spray drying using a Lab spray dryer, wherein the inlet air temperature is 40°C, to form microparticles. In a preferred embodiment, spray drying, optionally followed by lyophilization, fluid bed drying, or vacuum drying, is used to remove the solvents and the pore forming agent

In a preferred embodiment, the porous drug matrix is reconstituted with an aqueous medium and administered parenterally, such as intramuscularly, subcutaneously, or intravenously. Alternatively, the porous drug matrix can be further processed using standard techniques into tablets or capsules for oral administration or into rectal suppositories, delivered using a dry powder inhaler for pulmonary administration; or mixed/processed into a cream or ointment for topical administration.

An advantage of the porous drug matrix formulations is that they can be administered as a bolus, when the drug, such as paclitaxel, normally must be infused to avoid precipitation of the drug. By avoiding precipitation of drug *in vivo,* the formulations can also be administered intrarterially, intravenously, locally, intracranially, intrathecally, or, if appropriate, directly into a tumor. An additional advantage is the formulations can be administered in reduced volumes.

In one embodiment, the matrix further includes a pegylated excipient, such as pegylated phospholipid, with the drug. The pegylated excipient shields the drug from macrophage uptake, which prolong its half-life or enhance bioavailability of the drug.

### Brief Description of the Drawings

Figure 1 is a graph of the *in vitro* dissolution rate (percent dissolved versus time) for non-formulated prednisone and prednisone in porous matrix form.
Figure 2 is a graph of the *in vitro* dissolution rate (percent dissolved versus time) for non-formulated griseofulvin and griseofulvin in porous matrix form .
Figure 3 is a graph of the *in vitro* dissolution rate (percent dissolved versus time) for non-formulated nifedipine and nifedipine in porous matrix form.
Figure 4 is a graph of the *in vitro* dissolution rate (percent dissolved versus time) for non-formulated naproxen and naproxen in a porous matrix form.
Figure 5 is a graph of the *in vitro* dissolution rate (percent dissolved versus time) for non-formulated paclitaxel and paclitaxel in a porous matrix form.
Figure 6 is a graph of the *in vitro* dissolution rate (percent dissolved versus time) for various porous matrix forms of nifedipine.
Figure 7 is a graph of the *in vitro* dissolution rate (percent dissolved versus time) for various porous matrix forms of griseofulvin.
Figure 8 is a graph of nifedipine plasma levels versus time post intravenous administration of reconstituted nifedipine matrix in dogs.
Figure 9 shows the chemical structure of taxane compounds, including paclitaxel and docetaxel.

### Detailed Description of the Invention

The rate of dissolution of drugs can be enhanced by making the drug into a porous matrix form, substantially increasing the surface area of the drug available to contact aqueous biological fluids at the site of administration of the drug composition. The method for making the porous matrix of drug includes the steps of (a) dissolving a drug in a volatile solvent to form a drug solution, (b) combining at least one pore forming agent with the drug solution to form an emulsion, suspension, or second solution, and (c) removing the volatile solvent and pore forming agent from the emulsion, suspension, or second solution to yield the porous matrix of drug as defined in claim 1*.*

In a preferred embodiment, pharmaceutical compositions are provided for paclitaxel, or docetaxel, without the solubilizing agent CREMOPHOR™, or Polysorbate 80, respectively, wherein the pharmaceutical composition can be administered as a bolus. The compositions are porous dry powders, which upon the addition of an aqueous medium form a suspension ofnanoparticles and microparticles of the drug.

### I. Drug Matrix Compositions

The porous drug matrix is at least 1 to 95%, preferably at least about 10%, and more preferably between about 10 and 70%, drug by weight. The matrices also may contain hydrophilic excipients such as water soluble polymers or sugars, wetting agents such as surfactants, and tonicity agents.

The form of the drug matrix (drug powder) is critical to the dissolution rate. The matrix must contain microparticles of drug, which preferably have a mean diameter between about 0.1 and 5 µm, more preferably between about 0.1 and 5 µm. In one embodiment, the mean diameter of the microparticles is between about 1 and 5 µm. The average total surface area of the microparticles contained within the porous matrix, which typically is in the form of a dry powder, is 0.5 m²/mL or greater, preferably 0.9 m²/mL or greater. Total surface area values for the microparticles can be determined using standard Coulter Counter equipment and techniques.

The drug matrix must be sufficiently porous to yield, upon contact with an aqueous medium, microparticles having these parameters. Measurements useful in characterizing the porosity of the drug matrix are the bulk density or the transaxial pressure ("TAP") density of the dry porous matrix (dry powder) and the total surface area (sum of internal and external surface area) of the dry porous matrix. The TAP density preferably is less than or equal to 1.0 g/ml, more preferably less than 0.8 g/ml. This level of porosity of the matrix, characterized by density, provides sufficient surface area to enhance wetting of the dry porous matrix and enhance drug dissolution. The total surface area of the porous matrix can be measured, for example, by BET surface area analysis. In some embodiments, the total surface area of the porous matrix preferably is greater than 0.1 m²/g, more preferably greater than or equal to 0.2 m²/g. This level of total surface area provides sufficient surface area to enhance wetting of the dry porous matrix and enhance drug dissolution.

### 1. Drugs

A wide variety drugs are useful in the methods and compositions described herein. In a preferred embodiment, the drug is a low aqueous solubility drug. As used herein, the term "low aqueous solubility" means that the drug has a solubility of less than about 10 mg/mL, and preferably less than about 5 mg/mL, in aqueous media at approximately physiological temperatures and pH. As used herein, the term "drug" refers to chemical or biological molecules providing a therapeutic, diagnostic, or prophylactic effect *in vivo.* A particularly preferred class of drugs is taxanes.

### (i) Taxanes

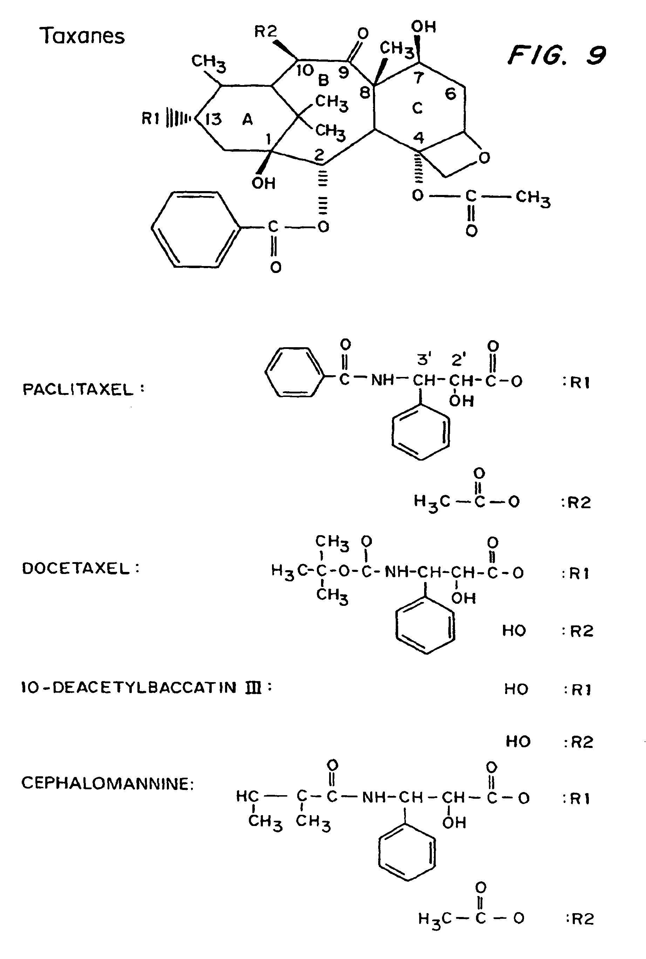

Taxanes are anticancer cytotoxics that stabilize cellular microtubules. Taxane compounds useful in the compositions and methods described herein include paclitaxel and docetaxel, as well as natural and synthetic analogs thereof which possess anticancer or anti-angiogenic activity. Paclitaxel and docetaxel have substantial activity, and one or both of these agents are widely accepted as components of therapy for advanced breast, lung, and ovarian carcinomas. Paclitaxel has recently been approved in the USA for the adjuvant treatment of early stage node-positive breast carcinoma. The chemical structure of taxanes, including paclitaxel and docetaxel, is shown in Figure 9.

### (ii) Other Drugs

Other drugs contemplated for use in the compositions described herein include the following categories and examples of drugs and alternative forms of these drugs such as alternative salt forms, free acid forms, free base forms, and hydrates:
analgesics/antipyretics (e.g., aspirin, acetaminophen, ibuprofen, naproxen sodium, buprenorphine, propoxyphene hydrochloride, propoxyphene napsylate, meperidine hydrochloride, hydromorphone hydrochloride, morphine, oxycodone, codeine, dihydrocodeine bitartrate, pentazocine, hydrocodone bitartrate, levorphanol, diflunisal, trolamine salicylate, nalbuphine hydrochloride, mefenamic acid, butorphanol, choline salicylate, butalbital, phenyltoloxamine citrate, diphenhydramine citrate, methotrimeprazine, cinnamedrine hydrochloride, and meprobamate);
antiasthamatics (e.g., ketotifen and traxanox);
antibiotics (e.g., neomycin, streptomycin, chloramphenicol, cephalosporin, ampicillin, penicillin, tetracycline, and ciprofloxacin);
antidepressants (e.g., nefopam, oxypertine, doxepin, amoxapine, trazodone, amitriptyline, maprotiline, phenelzine, desipramine, nortriptyline, tranylcypromine, fluoxetine, doxepin, imipramine, imipramine pamoate, isocarboxazid, trimipramine, and protriptyline);
antidiabetics (e.g., biguanides and sulfonylurea derivatives);
antifungal agents (e.g., griseofulvin, ketoconazole, itraconizole, amphotericin B, nystatin, and candicidin);
antihypertensive agents (e.g., propanolol, propafenone, oxyprenolol, nifedipine, reserpine, trimethaphan, phenoxybenzamine, pargyline hydrochloride, deserpidine, diazoxide, guanethidine monosulfate, minoxidil, rescinnamine, sodium nitroprusside, rauwolfia serpentina, alseroxylon, and phentolamine);
anti-inflammatories (e.g., (non-steroidal) indomethacin, ketoprofen, flurbiprofen, naproxen, ibuprofen, ramifenazone, piroxicam, (steroidal) cortisone, dexamethasone, fluazacort, celecoxib, rofecoxib, hydrocortisone, prednisolone, and prednisone);
antineoplastics (e.g., cyclophosphamide, actinomycin, bleomycin, daunorubicin, doxorubicin, epirubicin, mitomycin, methotrexate, fluorouracil, carboplatin, carmustine (BCNU), methyl-CCNU, cisplatin, etoposide, camptothecin and derivatives thereof, phenesterine, vinblastine, vincristine, tamoxifen, and piposulfan);
antianxiety agents (e.g., lorazepam, buspirone, prazepam, chlordiazepoxide, oxazepam, clorazepate dipotassium, diazepam, hydroxyzine pamoate, hydroxyzine hydrochloride, alprazolam, droperidol, halazepam, chlormezanone, and dantrolene);
immunosuppressive agents (e.g., cyclosporine, azathioprine, mizoribine, and FK506 (tacrolimus));
antimigraine agents (e.g., ergotamine, propanolol, isometheptene mucate, and dichloralphenazone);
sedatives/hypnotics (e.g., barbiturates such as pentobarbital, pentobarbital, and secobarbital; and benzodiazapines such as flurazepam hydrochloride, triazolam, and midazolam);
antianginal agents (e.g., beta-adrenergic blockers; calcium channel blockers such as nifedipine, and diltiazem; and nitrates such as nitroglycerin, isosorbide dinitrate, pentaerythritol tetranitrate, and erythrityl tetranitrate);
antipsychotic agents (e.g., haloperidol, loxapine succinate, loxapine hydrochloride, thioridazine, thioridazine hydrochloride, thiothixene, fluphenazine, fluphenazine decanoate, fluphenazine enanthate, trifluoperazine, chlorpromazine, perphenazine, lithium citrate, and prochlorperazine);
antimanic agents (e.g., lithium carbonate);
antiarrhythmics (e.g., bretylium tosylate, esmolol, verapamil, amiodarone, encainide, digoxin, digitoxin, mexiletine, disopyramide phosphate, procainamide, quinidine sulfate, quinidine gluconate, quinidine
polygalacturonate, flecainide acetate, tocainide, and lidocaine);
antiarthritic agents (e.g., phenylbutazone, sulindac, penicillamine, salsalate, piroxicain, ivathioprine, indomethacin, meclofenamate, gold sodium thiomalate, ketoprofen, auranofin, aurothioglucose, and tolmetin sodium);
antigout agents (e.g., colchicine, and allopurinol);
anticoagulants (e.g., heparin, heparin sodium, and warfarin sodium);
thrombolytic agents (e.g., urokinase, streptokinase, and alteplase);
antifibrinolytic agents (e.g., aminocaproic acid);
hemorheologic agents (e.g., pentoxifylline);
antiplatelet agents (e.g., aspirin);
anticonvulsants (e.g., valproic acid, divalproex sodium, phenytoin, phenytoin sodium, clonazepam, primidone, phenobarbitol, carbamazepine, amobarbital sodium, methsuximide, metharbital, mephobarbital, mephenytoin, phensuximide, paramethadione, ethotoin, phenacemide, secobarbitol sodium, clorazepate dipotassium, and trimethadione);
antiparkinson agents (e.g., ethosuximide);
antihistamines/antipruritics (e.g., hydroxyzine, diphenhydramine, chlorpheniramine, brompheniramine maleate, cyproheptadine hydrochloride, terfenadine, clemastine fumarate, triprolidine, carbinoxamine, diphenylpyraline, phenindamine, azatadine, tripelennamine, dexchlorpheniramine maleate, and methdilazine);
agents useful for calcium regulation (e.g., calcitonin, and parathyroid hormone);
antibacterial agents (e.g., amikacin sulfate, aztreonam, chloramphenicol, chloramphenicol palmitate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, and colistin sulfate);
antiviral agents (e.g., interferon alpha, beta or gamma, zidovudine, amantadine hydrochloride, ribavirin, and acyclovir);
antimicrobials (e.g., cephalosporins such as cefazolin sodium, cephradine, cefaclor, cephapirin sodium, ceftizoxime sodium; cefoperazone sodium, cefotetan disodium, cefuroxime axetil, refotaxime sodium, cefadroxil monohydrate, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, and cefuroxime sodium; penicillins such as ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G procaine, methicillin sodium, and nafcillin sodium; erythromycins such as erythromycin ethylsuccinate, erythromycin, erythromycin estolate, erythromycin lactobionate, erythromycin stearate, and erythromycin ethylsuccinate; and tetracyclines such as tetracycline hydrochloride, doxycycline hyclate, and minocycline hydrochloride, azithromycin, clarithromycin);
anti-infectives (e.g., GM-CSF);
bronchodilators (e.g., sympathomimetics such as epinephrine hydrochloride, metaproterenol sulfate, terbutaline sulfate, isoetharine, isoetharine mesylate, isoetharine hydrochloride, albuterol sulfate, albuterol, bitolterolmesylate, isoproterenol hydrochloride, terbutaline sulfate, epinephrine bitartrate, metaproterenol sulfate, epinephrine, and epinephrine bitartrate; anticholinergic agents such as ipratropium bromide; xanthines such as aminophylline, dyphylline, metaproterenol sulfate, and aminophylline; mast cell stabilizers such as cromolyn sodium; inhalant corticosteroids such as beclomethasone dipropionate (BDP), and beclomethasone dipropionate monohydrate; salbutamol; ipratropium bromide; budesonide; ketotifen; salmeterol; xinafoate; terbutaline sulfate; triamcinolone; theophylline; nedocromil sodium; metaproterenol sulfate; albuterol; flunisolide; fluticasone proprionate;
steroidal compounds and hormones (e.g., androgens such as danazol, testosterone cypionate, fluoxymesterone, ethyltestosterone, testosterone enathate, methyltestosterone, fluoxymesterone, and testosterone cypionate; estrogens such as estradiol, estropipate, and conjugated estrogens; progestins such as methoxyprogesterone acetate, and norethindrone acetate; corticosteroids such as triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate; and thyroid hormones such as levothyroxine sodium);
hypoglycemic agents (e.g., human insulin, purified beef insulin, purified pork insulin, glyburide, chlorpropamide, glipizide, tolbutamide, and tolazamide);
hypolipidemic agents (e.g., clofibrate, dextrothyroxine sodium, probucol, pravastitin, atorvastatin, lovastatin, and niacin);
proteins (e.g., DNase, alginase, superoxide dismutase, and lipase);
nucleic acids (e.g., sense or anti-sense nucleic acids encoding any therapeutically useful protein, including any of the proteins described herein);
agents useful for erythropoiesis stimulation (e.g., erythropoietin);
antiulcer/antireflux agents (e.g., famotidine, cimetidine, and ranitidine hydrochloride);
antinauseants/antiemetics (e.g., meclizine hydrochloride, nabilone, prochlorperazine, dimenhydrinate, promethazine hydrochloride, thiethylperazine, and scopolamine);
oil-soluble vitamins (e.g., vitamins A, D, E, K, and the like);
as well as other drugs such as mitotane, halonitrosoureas, anthrocyclines, and ellipticine. A description of these and other classes of useful drugs and a listing of species within each class can be found in Martindale, *The Extra Pharmacopoeia, 30th Ed.* (The Pharmaceutical Press, London 1993).

Examples of other drugs useful in the compositions and methods described herein include ceftriaxone, ketoconazole, ceftazidime, oxaprozin, albuterol, valacyclovir, urofollitropin, famciclovir, flutamide, enalapril, mefformin, itraconazole, buspirone, gabapentin, fosinopril, tramadol, acarbose, lorazepan, follitropin, glipizide, omeprazole, fluoxetine, lisinopril, tramsdol, levofloxacin, zafirlukast, interferon, growth hormone, interleukin, erythropoietin, granulocyte stimulating factor, nizatidine, bupropion, perindopril, erbumine, adenosine, alendronate, alprostadil, benazepril, betaxolol, bleomycin sulfate, dexfenfluramine, diltiazem, fentanyl, flecainid, gemcitabine, glatiramer acetate, granisetron, lamivudine, mangafodipir trisodium, mesalamine, metoprolol fumarate, metronidazole, miglitol, moexipril, monteleukast, octreotide acetate, olopatadine, paricalcitol, somatropin, sumatriptan succinate, tacrine, verapamil, nabumetone, trovafloxacin, dolasetron, zidovudine, finasteride, tobramycin, isradipine, tolcapone, enoxaparin, fluconazole, lansoprazole, terbinafine, pamidronate, didanosine, diclofenac, cisapride, venlafaxine, troglitazone, fluvastatin, losartan, imiglucerase, donepezil, olanzapine, valsartan, fexofenadine, calcitonin, and ipratropium bromide. These drugs are generally considered to be water soluble.

Preferred drugs include albuterol or albuterol sulfate, adapalene, doxazosin mesylate, mometasone furoate, ursodiol, amphotericin, enalapril maleate, felodipine, nefazodone hydrochloride, valrubicin, albendazole, conjugated estrogens, medroxyprogesterone acetate, nicardipine hydrochloride, zolpidem tartrate, amlodipine besylate, ethinyl estradiol, omeprazole, rubitecan, amlodipine besylate/ benazepril hydrochloride, etodolac, paroxetine hydrochloride, atovaquone, felodipine, podofilox, paricalcitol, betamethasone dipropionate, fentanyl, pramipexole dihydrochloride, Vitamin D₃ and related analogues, finasteride, quetiapine fumarate, alprostadil, candesartan, cilexetil, fluconazole, ritonavir, busulfan, carbamazepine, flumazenil, risperidone, carbemazepine, carbidopa, levodopa, ganciclovir, saquinavir, amprenavir, carboplatin, glyburide, sertraline hydrochloride, rofecoxib carvedilol, halobetasolproprionate, sildenafil citrate, celecoxib, chlorthalidone, imiquimod, simvastatin, ciprofloxacin, irinotecan hydrochloride, sparfloxacin, efavirenz, cisapride monohydrate, lansoprazole, tamsulosin hydrochloride, mofafinil, clarithromycin, letrozole, terbinafine hydrochloride, rosiglitazone maleate, diclofenac sodium, lomefloxacin hydrochloride, tirofiban hydrochloride, telmisartan, diazapam, loratadine, toremifene citrate, thalidomide, dinoprostone, mefloquine hydrochloride, trandolapril, docetaxel, mitoxantrone hydrochloride, tretinoin, etodolac, triamcinolone acetate, estradiol, ursodiol, nelfinavir mesylate, indinavir, beclomethasone dipropionate, oxaprozin, flutamide, famotidine, nifedipine, prednisone, cefuroxime, lorazepam, digoxin, lovastatin, griseofulvin, naproxen, ibuprofen, isotretinoin, tamoxifen citrate, nimodipine, amiodarone, and alprazolam.

### 2. Excipients

The matrices may contain hydrophilic excipients such as water soluble polymers or sugars which can serve as bulking agents or as wetting agents, wetting agents such as surfactants or sugars, and tonicity agents. Upon contact with an aqueous medium, water penetrates through the highly porous matrix to dissolve the water soluble excipients in the matrix. In the case of low aqueous solubility drugs, a suspension of drug particles in the aqueous medium remains. The total surface area of the resultant low aqueous solubility drug microparticles is increased relative to the unprocessed drug and the dissolution rate of the drug is increased.

One of skill in the art can select appropriate excipients for use in the drug matrix compositions, considering a variety of factors, such as the drug to be administered, the route of administration, the dosage, and the preferred dissolution rate. For example, the excipients can function as bulking agents, release-modifiers, wetting agents, tonicity agents, or combinations thereof. Preferred excipients include hydrophilic polymers, wetting agents, and sugars. The amount of excipient in the drug matrix is less than about 95%, more preferably less than about 80%, by weight of the drug matrix.

The hydrophilic excipients, wetting agents, and tonicity agents may be added to the drug solution, the pore forming agent, or both, during production of the matrix.

### (i) Hydrophilic Polymers

The polymers that can be used in the drug matrices described herein include both synthetic and natural polymers, either non-biodegradable or biodegradable. Representative synthetic polymers include polyethylene glycol ("PEG"), polyvinyl pyrrolidone, polymethacrylates, polylysine, poloxamers, polyvinyl alcohol, polyacrylic acid, polyethylene oxide, and polyethyoxazoline. Representative natural polymers include albumin, alginate, gelatin, acacia, chitosan, cellulose dextran, ficoll, starch, hydroxyethyl cellulose, hydroxypropyl cellulose, hydroxy-propylmethyl cellulose, hyaluronic acid, carboxyethyl cellulose, carboxymethyl cellulose, deacetylated chitosan, dextran sulfate, and derivatives thereof. Preferred hydrophilic polymers include PEG, polyvinyl pyrrolidone, poloxamers, hydroxypropyl cellulose, and hydroxyethyl cellulose.

The hydrophilic polymer selected for use in a particular drug matrix formulation is based on a variety of factors, such as the polymer molecular weight, polymer hydrophilicity, and polymer inherent viscosity. The hydrophilic polymer can be used as a bulking agent or as a wetting agent.

### (ii) Sugars

Representative sugars that can be used in the drug matrices include mannitol, sorbitol, xylitol, glucitol, ducitol, inosittol, arabinitol, arabitol, galactitol, iditol, allitol, fructose, sorbose, glucose, xylose, trehalose, allose, dextrose, altrose, gulose, idose, galactose, talose, ribose, arabinose, xylose, lyxose, sucrose, maltose, lactose, lactulose, fucose, rhamnose, melezitose, maltotriose, and raffinose. Preferred sugars include mannitol, lactose, sucrose, sorbitol, trehalose, glucose, and are adjusted to provide osmolality if administered parenterally or to provide wetting of the porous drug matrix or the drug microparticles within the matrix.

### (iii) Wetting Agents

Wetting agents can be used to facilitate water ingress into the matrix and wetting of the drug particles in order to facilitate dissolution. Representative examples of wetting agents include gelatin, casein, lecithin (phosphatides), gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters (e.g., TWEEN™s), polyethylene glycols, polyoxyethylene stearates, colloidal silicon dioxide, phosphates, sodium dodecylsulfate, carboxymethylcellulose calcium, carboxymethylcellulose sodium, methylcellulose, hydroxyethylcellulose, hydroxy propylcellulose, hydroxypropylmethylcellulose phthlate, noncrystalline cellulose, magnesium aluminum silicate, triethanolamine, polyvinyl alcohol, and polyvinylpyrrolidone (PVP). Tyloxapol (a nonionic liquid polymer of the alkyl aryl polyether alcohol type, also known as superinone or triton) is another useful wetting agent. Most of these wetting agents are known pharmaceutical excipients and are described in detail in the Handbook of Pharmaceutical Excipients, published jointly by the American Pharmaceutical Association and The Pharmaceutical Society of Great Britain (The Pharmaceutical Press, 1986).

Preferred wetting agents include polyvinylpyrrolidone, polyethylene glycol, tyloxapol, poloxamers such as PLURONIC™ F68, F127, and F108, which are block copolymers of ethylene oxide and propylene oxide, and polyxamines such as TETRONIC™ 908 (also known as POLOXAMINE™ 908), which is a tetrafunctional block copolymer derived from sequential addition of propylene oxide and ethylene oxide to ethylenediamine (available from BASF), dextran, lecithin, dialkylesters of sodium sulfosuccinic acid such as AEROSOL™ OT, which is a dioctyl ester of sodium sulfosuccinic acid (available from American Cyanimid), DUPONOL™ P, which is a sodium lauryl sulfate (available from DuPont), TRITON™ X-200, which is an alkyl aryl polyether sulfonate (available from Rohm and Haas), TWEEN™ 20 and TWEEN™ 80, which are polyoxyethylene sorbitan fatty acid esters (available from ICI Specialty Chemicals), Carbowax 3550 and 934, which are polyethylene glycols (available from Union Carbide), Crodesta F-110, which is a mixture of sucrose stearate and sucrose distearate, and Crodesta SL-40 (both available from Croda Inc.), and SA90HCO, which is C₁₈H₃₇CH₂(CON(CH₃)CH₂(CHOH)₄CH₂OH)₂.

Wetting agents which have been found to be particularly useful include Tetronic 908, the Tweens, Pluronic F-68 and polyvinylpyrrolidone. Other useful wetting agents include decanoyl-N-methylglucamide; n-decyl-β-D-glucopyranoside; n-decyl-β-D-maltopyranoside; n-dodecyl-β-D-glucopyranoside; n-dodecyl β-D-maltoside; heptanoyl-N-methylglucamide; n-heptyl-β-D-glucopyranoside; n-heptyl-β-D-thioglucoside; n-hexyl-β-D-glucopyranoside; nonanoyl-N-methylglucamide; n-noyl-β-D-glucopyranoside; octanoyl-N-methylglucamide; n-octyl-β-D-glucopyranoside; and octyl-β-D-thioglucopyranoside. Another preferred wetting agent is p-isononylphenoxypoly(glycidol), also known as Olin-10G or Surfactant 10-G (commercially available as 10G from Olin Chemicals). Two or more wetting agents can be used in combination.

### (iv) Tonicity or Osmolality Agents

The porous drug matrices may include one or more tonicity agents, such as salts (e.g., as sodium chloride or potassium chloride) or sugars (such as mannitol, dextrose, sucrose, or trehalose) to adjust a hypotonic solution of a drug to isotonic so that the drug, when in solution, is physiologically compatible with the cells of the body tissue of the patient. The type and amount of tonicity agent can be selected by one of skill in the art using known techniques.

### (v) Pegylated Excipients

In one embodiment, the matrix further includes a pegylated excipient. Such pegylated excipients include, but are not limited to, pegylated phospholipids, pegylated proteins, pegylated peptides, pegylated sugars, pegylated polysaccharides, pegylated block copolymers with one of the blocks being PEG, and pegylated hydrophobic compounds such as pegylated cholesterol. The pegylated excipient beneficially envelops or shields the drug from macrophage uptake, which prolongs its half-life or enhances bioavailability of the drug.

Representative examples of pegylated phospholipids include 1,2-diacyl-sn-glycero-3-phosphoethanolamine-N-[Poly(ethylene glycol) 2000] ("PEG 2000 PE") and 1,2-diacyl-sn-glycero-3-phosphoethanolamine-N-[Poly(ethylene glycol) 5000] ("PEG 5000 PE"), where the acyl group is selected, for example, from dimyristoyl, dipalmitoyl, distearoyl, diolcoyl, and 1-palnutoyl-2-oleoyl.

Other polyalkyleneoxides can be used in place of the polyethylene.

### II. Volatile Solvents

The choice of solvent depends on the drug. The solvent is an organic solvent that is volatile, has a relatively low boiling point, or can be removed under vacuum, and which is acceptable for administration to humans in trace amounts. Representative solvents include acetic acid, acetaldehyde dimethyl acetal, acetone, acetonitrile, chloroform, chlorofluorocarbons, dichloromethane, dipropyl ether, diisopropyl ether, N,N-dimethlyformamide (DMF), foramide, demethyl sulfoxide (DMSO), dioxane, ethanol, ethyl acetate, ethyl formate, ethyl vinyl ether, methyl ethyl ketone (MEK), glycerol, heptane, hexane, isopropanol, methanol, isopropanol, butanol, triethylamine, nitromethane, octane, pentane, tetrahydrofuran (THF), toluene, 1,1,1-trichloroethane, 1,1,2-trichloroethylene, xylene, and combinations thereof. In general, the drug is dissolved in the volatile solvent to form a drug solution having a concentration of between 0.01 and 80% weight to volume (w/v), more preferably between 0.025 and 30% (w/v).

Aqueous solvents or mixtures of aqueous and organic solvents, such as water-alcohol mixtures, can be used to dissolve the drug.

### III. Pore Forming Agents

Pore forming agents are volatile materials that are used during the process to create porosity in the resultant matrix. The pore forming agent a volatilizable solid.

### Solid Pore Forming Agent

The solid pore forming agent must be volatilizable under processing conditions which do not harm the drug compositions. The solid pore forming agent can be (i) dissolved in the drug solution, (ii) dissolved in a solvent which is not miscible with the drug solvent to form a solution which is then emulsified with the drug solution, or (iii) added as solid particulates to the drug solution. The solution, emulsion, or suspension of the pore forming agent in the drug solution then is further processed to remove the drug solvent, the pore forming agent, and, if appropriate, the solvent-for the pore forming agent simultaneously or sequentially using evaporation, spray drying, fluid bed drying, lyophilization, vacuum drying, or a combination of these techniques.

In a preferred embodiment, the solid pore forming agent is a volatile salt, such as salts of volatile bases combined with volatile acids. Volatile salts are materials that can transform from a solid or liquid to a gaseous state using added heat and/or vacuum. Examples of volatile bases include ammonia, methylamine, ethylamine, dimethylamine, diethylamine, methylethylamine, trimethylamine, triethylamine, and pyridine. Examples of volatile acids include carbonic acid, hydrochloric acid, hydrobromic acid, hydroiodic acid, formic acid, acetic acid, propionic acid, butyric acid, and benzoic acid. Preferred volatile salts include ammonium bicarbonate, ammonium acetate, ammonium chloride, ammonium benzoate and mixtures thereof.

Other examples of solid pore forming agents include iodine, phenol, benzoic acid (as acid not as salt), and naphthalene.

The solid pore forming agent is used in an amount between 0.5 and 1000% (w/w), preferably between 10 and 600% (w/w), and more preferably between 1 and 100% (w/w), of the drug.

### IV. Method of Making the Porous Drug Matrix

The porous drug matrices preferably are made by (i) dissolving a drug, preferably one having low aqueous solubility, in a volatile solvent to form a drug solution, (ii) combining at least one pore forming agent with the drug solution to form an emulsion, suspension, or second solution, and (iii) removing the volatile solvent and pore forming agent from the emulsion, suspension, or second solution as defined in appended claim 1. In a preferred embodiment, spray drying, optionally followed by lyophilization or vacuum drying, is used to remove the solvents and the pore forming agent. The removal of the pore forming agent can be conducted simultaneously with or following removal of enough solvent to solidify the droplets. Production can be carried out using continuous, batch, or semi-continuous processes.

First, the selected drug is dissolved in an appropriate solvent. The concentration of the drug in the resulting drug solution typically is between about 0.01 and 80% (w/v), preferably between about 0.025 and 30% (w/v).

Next, the drug solution is combined, typically under mixing conditions, with the pore forming agent or solution thereof. If a liquid pore forming agent is used, it is first emulsified with the drug solution to form droplets of pore forming agent dispersed throughout the drug solution. If a solid pore forming agent is used, it is dissolved either directly in the drug solution to form a solution of drug/pore forming agent, or it is first dissolved in a second solvent. If the second solvent is immiscible with the drug solvent, the solution of the pore forming agent is emulsified with the drug solution to form droplets of the pore forming agent solution dispersed throughout the drug solution. If the second solvent is miscible with the drug solution, the two solutions are mixed to form a single drug solution. A solid pore forming agent alternatively can be added directly to the drug solution as solid particulates, preferably between about 100 nm and 10 µm in size, to form a suspension of pore forming agent in the drug solution. Subsequently, the solid pore forming agent particle size can be reduced by further processing the resulting suspension, for example, using homogenization or sonication techniques known in the art.

Then, the solution, emulsion, or suspension is further processed to remove the drug solvent and the pore forming agent simultaneously or sequentially, using evaporation, spray drying, fluid bed drying, lyophilization, vacuum drying, or a combination of these techniques. In a preferred embodiment, the solution, emulsion, or suspension is spray-dried. As used herein, "spray dry" means to atomize the solution, emulsion, or suspension to form a fine mist of droplets (of drug solution having solid or liquid pore forming agent dispersed throughout), which immediately enter a drying chamber (e.g., a vessel, tank, tubing, or coil) where they contact a drying gas. The solvent and pore forming agents evaporate from the droplets into the drying gas to solidify the droplets, simultaneously forming pores throughout the solid. The solid (typically in a powder, particulate form) then is separated from the drying gas and collected.

The temperature of the inlet and outlet ports of the drying chamber, as well as the flow rates of the feed solution, atomization gas, and drying gas, can be controlled to produce the desired products. In a particularly preferred embodiment, the spray drying methods described in U.S. Patent No. 5,853,698 to Straub et al. are adapted to make the drug matrices.

The drug present in the solids or powder produced may be in a crystalline or an amorphous state, or may be mixture of such states. The state generally depends on how the droplets are dried and the excipients present.

### Emulsion Stabilization

In embodiments in which at least one pore forming agent is combined with the drug solution to form an emulsion, a surfactant or emulsifying agent can be added to enhance the stability of the emulsion. A variety of surfactants may be incorporated in this process, preferably to an amount between 0.1 and 5% by weight. Exemplary emulsifiers or surfactants which may be used include most physiologically acceptable emulsifiers, for instance egg lecithin or soya bean lecithin, or synthetic lecithins such as saturated synthetic lecithins, for example, dimyristoyl phosphatidyl choline, dipalmitoyl phosphatidyl choline or distearoyl phosphatidyl choline or unsaturated synthetic lecithins, such as dioleyl phosphatidyl choline or dilinoleyl phosphatidyl choline. Other hydrophobic or amphipathic compounds can be used in place of the phospholipid, for example, cholesterol. Emulsifiers also include surfactants such as free fatty acids, esters of fatty acids with polyoxyalkylene compounds like polyoxpropylene glycol and polyoxyethylene glycol; ethers of fatty alcohols with polyoxyalkylene glycols; esters of fatty acids with polyoxyalkylated sorbitan; soaps; glycerol-polyalkylene stearate; glycerol-polyoxyethylene ricinoleate; homo- and co-polymers of polyalkylene glycols; polyethoxylated soya-oil and castor oil as well as hydrogenated derivatives; ethers and esters of sucrose or other carbohydrates with fatty acids, fatty alcohols, these being optionally polyoxyalkylated; mono-, di- and tri-glycerides of saturated or unsaturated fatty acids, glycerides of soya-oil and sucrose.

Other emulsifiers include natural and synthetic forms of bile salts or bile acids, both conjugated with amino acids and unconjugated such as taurodeoxycholate and cholic acid.

### V. Porous Drug Matrix Applications

The porous drug matrices described herein are useful in formulations for administration to a patient in need of the drug. As used herein, "patient" refers to animals, including mammals, preferably humans. The formulations deliver a therapeutically or prophylactically effective amount of the drug to the patient.

In a preferred embodiment, paclitaxel or docetaxel matrices are used in formulations for administration to a patient in need thereof. Pharmaceutical compositions of these drugs preferably are provided without CREMOPHOR™, Polysorbate 80, or other solubilizing agents, for bolus administration. The compositions are porous dry powders which upon the addition of an aqueous medium form a suspension of nanoparticles and microparticles of the drug.

The porous matrices, or formulations thereof, are suitable for administration of drug by a variety of routes, for example, parenteral, mucosal, oral, topical/transdermal administration, for local, regional, or systemic effect. Examples of parenteral routes include intraveneous, intraarterial, intracardiac, intrathecal, intraosseous, intraarticular, intrasynovial, intracutaneous, subcutaneous, and intramuscular administration. Examples of mucosal routes include pulmonary (intrarespiratory), buccal, sublingual, intranasal, rectal, and vaginal administration. The porous matrices also can be formulated for intraocular, conjunctival, aural, urethral, intracranial, intralesional, and intratumoral administration.

In a preferred embodiment, the drug matrix is in the form of powder, which can be reconstituted with an aqueous medium, such as physiological saline, and administered parenterally, such as intramuscularly, subcutaneously, or intravenously. An advantage of the formulations described herein is that they can be used to convert drugs which must be infused (e.g., to avoid precipitation of the drug following bolus injection) to a bolus formulation, avoiding unacceptable precipitation of drug *in vivo* or for local delivery.

Alternatively, the matrix can be further processed using standard techniques into tablets or capsules for oral administration, into rectal suppositories, into a dry powder inhaler for pulmonary administration, or mixed/processed into a cream or ointment for topical administration. These standard techniques are described, for example, in Ansel, et al., *"Pharmaceutical Dosage Forms and Drug Delivery Systems*," 6^{th} Ed., (Williams & Wilkins 1995).

The present invention will be further understood with reference to the following non-limiting examples.

### Overview

Examples 1-10 demonstrate production of porous drug matrices using different pore forming agents, different drugs, and different solvents. Examples 1-8 use emulsion formulations to produce the matrices, whereas Examples 9 and 10 use solution formulations to produce the matrices. Examples 11-13 describe the analyses which were used to characterize the porous drug matrices produced in Examples 1-10. These characteristics include density, drug integrity, and dissolution properties.

Example 14 describes particle size analysis and surface area analysis of low water solubility drug particles incorporated into the porous drug matrices. Examples 15-17 describe experiments demonstrating the increased internal surface area of porous drug matrices produced with pore forming agents. Examples 18-21 describe experiments demonstrating the advantage or need to include a wetting agent as a component of the porous drug matrices. Example 22 describes an experiment demonstrating the administration of porous drug matrices as an intravenous bolus. Examples 23 and 24 describe the production of porous drug matrices produced with pore forming agents and pegylated phospholipids.

### Materials and Equipment

The following materials and equipment were used in the examples. PEG 3350, PEG 8000, polyvinylpyrrolidone K-15, nifedipine, naproxen, prednisone, SPAN™ 40, lecithin, TWEEN™ 80, PLURONIC™ F127, ammonium chloride, ammonium bicarbonate, and ammonium acetate were obtained from Spectrum Chemicals (Gardena, CA). Griseofulvin was obtained from Aldrich Chemicals (Milwaukee, WI). Paclitaxel was obtained from Hauser (Boulder, CO). 1,2-Dimyristoyl-sn-Glycero-3-Phosphoethanolamine-N-[Poly(ethylene glycol)-5000] (PEG 5000 PE) and 1,2-Dimyristoyl-sn-Glycero-3-Phosphoethanolamine-N-[Poly(ethylene glycol)-2000] (PEG 2000 PE) were obtained from Avanti Polar Lipids Inc. (Alabaster, AL). Methylene chloride was obtained from EM Science (Gibbstown, NJ). All emulsions were produced using a Virtis IQ² homogenizer (Virtis, Gardiner, NY). Formulations were spray dried on a benchtop spray dryer using an air atomizing nozzle.

### Example 1: Production of a Porous Prednisone Matrix Using Ammonium Bicarbonate as a Pore Forming Agent with SPAN™ 40 and PEG 8000 as Wetting Agents

5.46 g of PEG 8000, 0.545 g of prednisone, and 0.055 g of SPAN™ 40 were dissolved in 182 mL of methylene chloride. An aqueous solution was prepared by dissolving 3.27 g of ammonium bicarbonate in 18.2 mL of deionized (DI) water. The aqueous solution was added to the organic solution (phase ratio 1:10) and homogenized for 5 minutes at 16,000 RPM. The resulting emulsion was spray dried on a benchtop spray dryer using an air-atomizing nozzle and nitrogen as the drying gas. Spray drying process conditions were 20 mL/min solution flow rate, 60 kg/hr drying gas rate, and 36 °C outlet temperature.

### Example 2: Production of a Porous Prednisone Matrix Using Ammonium Bicarbonate as a Pore Forming Agent with PEG 8000, TWEEN™ 80, Lecithin as Wetting Agents

5.46 g of PEG 8000, 0.545 g of prednisone, 0.003 g of TWEEN™ 80, and 0.003 g of lecithin were dissolved in 182 mL of methylene chloride. An aqueous solution was prepared as described in Example 1. The aqueous solution was added to the organic solution (phase ratio 1:10) and homogenized for 15 minutes as described in Example 1. The resulting emulsion was spray dried as described in Example 1 using process conditions of 20 mL/min solution flow rate, 60 kg/hr drying gas rate, and 35 °C outlet temperature.

### Example 3: Production of a Porous Prednisone Matrix Using Ammonium Acetate as a Pore Forming Agent, and PEG 8000, TWEEN™ 80, and Lecithin as Wetting Agents

A prednisone-loaded organic solution was prepared as described in Example 2. An aqueous solution was prepared by dissolving 3.27 g of ammonium acetate in 18.2 mL of DI water. The aqueous and organic solutions were homogenized and spray dried as described in Example 2.

### Example 4: Production of a Porous Prednisone Matrix Using Ammonium Chloride as a Pore Forming Agent, and PEG 8000, TWEEN™ 80, and Lecithin as Wetting Agents

A prednisone-loaded organic solution was prepared as described in Example 2. An aqueous solution was prepared by dissolving 3.27 g of ammonium chloride in 18.2 mL of DI water. The aqueous and organic solutions were homogenized as described in Example 1. The resulting emulsion was spray dried as described in Example 2.

### Example 5: Production of a Porous Griseofulvin Matrix Using Ammonium Bicarbonate as a Pore Forming Agent, and PEG 3350, TWEEN™ 80, and Lecithin as Wetting Agents

9.09 g of PEG 3350, 4.55 g of griseofulvin, 0.01 g of TWEEN™ 80, and 0.01 g of lecithin were dissolved in 182 mL of methylene chloride. An aqueous solution was prepared by dissolving 3.27 g of ammonium bicarbonate and 1.09 g of PEG 3350 in 18.2 mL of DI water. The aqueous and organic solutions were homogenized as described in Example 1. The resulting emulsion was spray dried as described in Example 1 using process conditions of 20 ml/min solution flow rate, 80 kg/hr drying gas rate, and 12 °C outlet temperature.

### Example 6: Production of a Porous Nifedipine Matrix Using Ammonium Bicarbonate as a Pore Forming Agent, and PEG 3350 and Lecithin as Wetting Agents

9.09 g of PEG 3350, 2.27 g of nifedipine, and 0.009 g of lecithin were dissolved in 182 mL of methylene chloride. An aqueous solution was prepared by dissolving 3.27 g of ammonium bicarbonate in 18.2 mL of DI water. The aqueous and organic solutions were homogenized in described in Example 1. The resulting emulsion was spray dried as described in Example 1 using process conditions of 20 ml/min solution flow rate, 60 kg/hr drying gas rate, and 20 °C outlet temperature.

### Example 7: Production of a Porous Naproxen Matrix Using Ammonium Chloride as a Pore Forming Agent, and PEG 3350 and Lecithin as Wetting Agents

A naproxen-loaded organic solution was prepared by dissolving 10.91 g of PEG 3350,2.73 g of naproxen, and 0.109 g of lecithin in 182 mL of methylene chloride. An aqueous solution was prepared as described in Example 4. The aqueous and organic solutions were homogenized as described in Example 1, and the resulting emulsion was spray dried using process conditions of 20 ml/min solution flow rate, 100 kg/hr drying gas rate, and 20 °C outlet temperature.

### Example 8: Production of a Porous Paclitaxel Matrix Using Ammonium Bicarbonate as a Pore Forming Agent, and PEG 3350 and Lecithin as Wetting Agents

A paclitaxel-loaded organic solution was prepared by dissolving 3.0 g of paclitaxel, 15.0 g of PEG 3350, and 15.7 mg of lecithin in 100 mL of methylene chloride. An aqueous solution composed of 1.8 g of ammonium bicarbonate and 0.6 g of PEG 3350 in 10 mL of DI water was added to the organic solution (phase ratio 1:10). The mixture was homogenized for 5 minutes at 16,000 RPM. The resulting emulsion was spray dried using process conditions of 10 mL/min solution flow rate, 60 kg/hr drying gas rate, and 25 °C outlet temperature.

### Example 9: Production of a Porous Nifedipine Matrix Using Ammonium Bicarbonate as a Pore Forming Agent, PEG 3350 and TWEEN™ 80 as Wetting Agents, Polyvinylpyrrolidone as a Bulking Agent, and Ethanol as a Solvent

A nifedipine-loaded organic solution was prepared by dissolving 0.76 g of nifedipine, 0.28 g of PEG 3350, and 2.72 g of polyvinylpyrrolidone K-15 in 170 mL of ethanol. An aqueous solution composed of 1.62 g of ammonium bicarbonate and 3 mg of TWEEN™ 80 in 30 mL of DI water was added to the ethanol solution and mixed. The resulting solution was spray dried using process conditions of 20 mL/min solution flow rate, 100 kg/hr drying gas rate, and 36 °C outlet temperature.

### Example 10: Production of a Porous Nifedipine Matrix Using Ammonium Bicarbonate as a Pore Forming Agent, PEG 3350 and PLURONIC™ F127 as Wetting Agents, Polyvinylpyrrolidone as a Bulking Agent, and Ethanol as a Solvent

A nifedipine-loaded organic solution was prepared by dissolving 0.76 g of nifedipine, 0.28 g of PEG 3350, and 2.72 g of polyvinylpyrrolidone K-15 in 170 mL of ethanol. An aqueous solution composed of 1.62 g of ammonium bicarbonate and 3 mg of PLURONIC™ F127 in 30 mL of DI water was added to the ethanol solution and mixed. The resulting solution was spray dried using process conditions of 20 mL/min solution flow rate, 100 kg/hr drying gas rate, and 36 °C outlet temperature.

### Example 11: In Vitro Dissolution of Porous Drug Matrices

The *in vitro* dissolution rates of the powders produced in Examples 1-10 were compared to the dissolution rates of the bulk drug of interest.

### Analytical Method

All dissolution studies were conducted in PBS (phosphate buffered saline) at room temperature in a glass beaker using overhead mixing. The mixer used was an IKARW16 Basic Mixer with a R1342 impeller shaft running at stirring rate 5. Samples were removed via pipet, filtered through 0.22 micron CA syringe filter, and then analyzed. UV-vis spectroscopy was conducted on an Hewlett Packard Model 8453. Dissolution curves are presented as percent of complete dissolution.

For griseofulvin, PBS (600 mL) was added to an appropriate amount of material being tested to contain 2.4 mg of griseofulvin. UV analysis was performed at 291 nm.

For naproxen, PBS (100 mL) was added to an appropriate amount of material being tested to contain 100 mg of naproxen. All vessels containing naproxen as a solid or as a solution were protected from light. UV analysis was performed at 332 nm.

For nifedipine, PBS (600 mL) was added to an appropriate amount of material being tested to contain 2.4 mg of nifedipine. All vessels containing nifedipine as a solid or in solution were protected from light. UV analysis was performed at 237 nm.

For prednisone, PBS (250 mL) was added to an appropriate amount of material being tested to contain 5 mg of prednisone. UV analysis was performed at 244 nm.

For paclitaxel, studies were conducted in PBS containing 0.08% TWEEN™ 80 (T80/PBS). T80/PBS (10 mL) was added to an appropriate amount of material being tested to contain 5 mg of paclitaxel in a 15 mL polypropylene conical tube, and the suspension was vortexed for 3-4 minutes. The suspension (0.25 mL) was then added to 250 mL of T80/PBS in a 600 mL glass beaker for dissolution analysis. HPLC analysis was performed directly on the filtered aqueous solutions using the paclitaxel HPLC method described in Example 13.

### Results

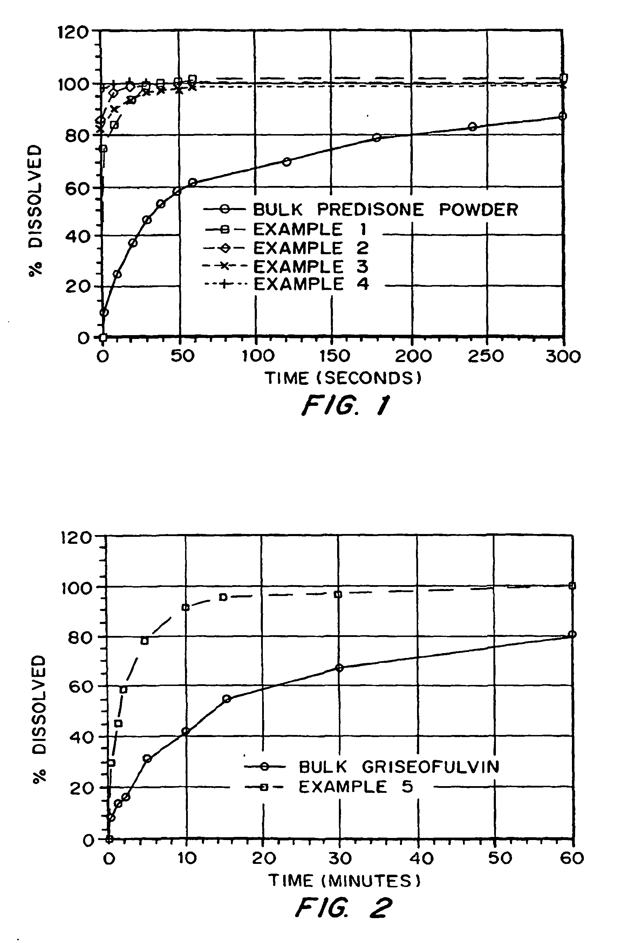

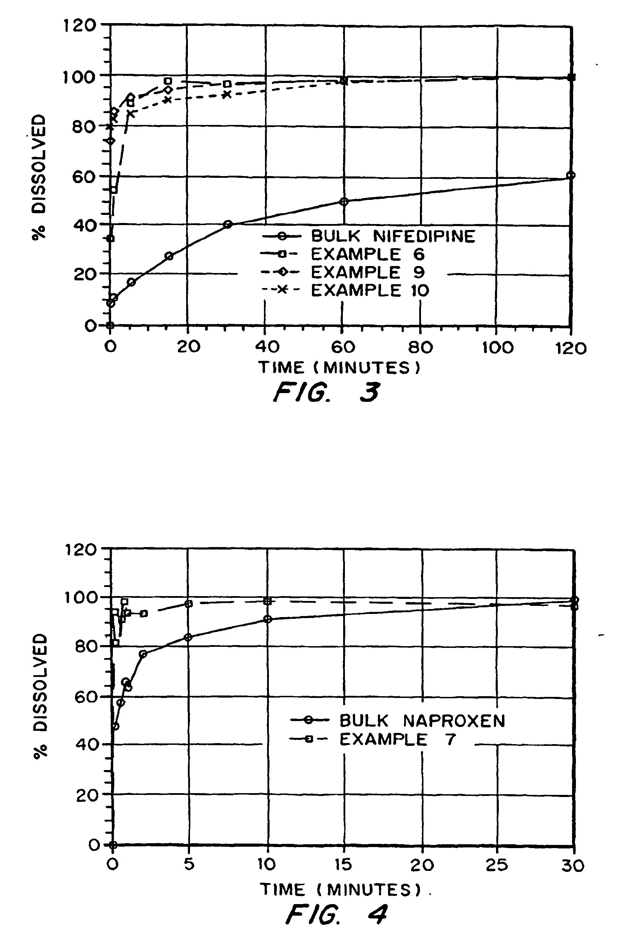

The *in vitro* dissolution rates of the porous drug matrices produced in examples 1-10 are provided in Figures 1-6. The *in vitro* dissolution of the porous drug matrices are compared to the bulk drug of interest. In all cases, the time for 80% dissolution of the porous drug matrices is 4-50 times shorter than the time for 80% of the bulk drug to dissolve. The rate of dissolution which is approximated as the slope of the curve is 10 to 1400 times greater for the porous drug matrices of Examples 1-10 as compared to the specific bulk drug of interest.

### Example 12: Density of Porous Drug Matrices

The densities of the dry powder produced in Examples 1-7 are summarized in Table 1. Density was measured using Transaxial Pressure ("TAP") with a Micromeritics GeoPyc 1360 using a consolidation force of 8 Newtons. The matrices are less dense than the starting bulk drug in all cases, indicating that the porous drug matrices are more porous than the commercially available bulk drug.

**Table 1:**

| **Particle Density Analysis** | |
|---|---|
| **Material** | **Density (g/mL)** |
| Prednisone Bulk | 0.68 |
| Example 1 | 0.48 |
| Example 2 | 0.55 |
| Example 3 | 0.51 |
| Example 4 | 0.49 |
| Griseofulvin Bulk | 0.80 |
| Example 5 | 0.55 |
| Nifedipine Bulk | 1.01 |
| Example 6 | 0.56 |
| Naproxen Bulk | 0.69 |
| Example 7 | 0.58 |

### Example 13: Integrity of the Drug in Porous Drug Matrices

### Analytical Method

Drug integrity post processing was assessed by High Pressure Liquid Chromatography ("HPLC") (Hewlett Packard Series 1100 HPLC). USP chromatography conditions were used for prednisone, naproxen, nifedipine, and griseofulvin. Vessels and vials containing naproxen or nifedipine solutions were protected from light. For paclitaxel, the chromatographic conditions included a Nucleosil column (5 :m, C18, 100A, 250 x 4.6 mm), a mobile phase of 2 mM H₃PO₄/Acetonitrile (2:3) at a flow rate of 1.5 mL,/min, UV detection at 227 nm, and a run time of 25 min.

### Results

The integrities of the drugs following the processing in Examples 1-9 are shown in Table 2 as purities. The process of forming the drug into porous matrices does not appear to alter the purity of the drug.

**Table 2:**

| **Drug Integrity Analysis** | |
|---|---|
| **Material** | **Purity (%)** |
| Prednisone Powder | 100 |
| Example 1 | 99.8 |
| Example 2 | 99.8 |
| Example 3 | 99.8 |
| Example 4 | 99.8 |
| Griseofulvin Bulk | 95.7 |
| Example 5 | 95.7 |
| Nifedipine Bulk | 100 |
| Example 6 | 100 |
| Example 9 | 100 |
| Example 10 | 100 |
| Naproxen Bulk | 100 |
| Example 7 | 100 |
| Paclitaxel Bulk | 100 |
| Example 8 | 100 |

### Example 14: Particle Size Analysis and Surface Area Analysis of Drug Particles in Wetted Porous Drug Matrices

### Analytical Methods

Particle size analysis was performed using the Coulter Multisizer II with a 50 micron aperture using siphon mode. Electrolyte was pre-saturated with the drug of interest, and filtered through a 0.22 micron filter prior to addition of lots for analysis to ensure that no portion of the drug within the lot would dissolve during the analysis.

### Results

The mean particle size and total surface area of the drug particles generated when the porous drug matrices produced in Examples 1-7 were reconstituted in aqueous media are summarized in Table 3.

**Table 3:**

| **Particle Size and Surface Area Analysis** | | |
|---|---|---|
| **Material** | **Size** **(microns)** | **Surface Area** **(m²/mL of microparticles)** |
| Prednisone Powder | 2.07 | 1.43 |
| Example 1 | 1.58 | 1.66 |
| Example 2 | 1.39 | 2.53 |
| Example 3 | 1.39 | 3.02 |
| Example 4 | 1.24 | 3.36 |
| Griseofulvin Bulk | 2.42 | 0.88 |
| Example 5 | 2.16 | 1.28 |
| Nifedipine Bulk | 2.64 | 0.57 |
| Example 6 | 1.78 | 1.98 |
| Naproxen Bulk | 2.89 | 0.66 |
| Example 7 | 1.34 | 2.79 |

In all cases, the particle size of the drug particles which resulted from wetting of the porous drug matrices was reduced relative to the starting bulk material by 10 to 54%, and the total surface area of the particles was increased relative to the starting bulk drug by approximately 16-320%.

### Example 15: Nifedipine Drug Matrices Containing a Wetting Agent Produced With and Without a Pore Forming Agent

A nifedipine/PEG solution was prepared by dissolving 2.0 g of nifedipine, 8.0 g of PEG 3350, and 8 mg of lecithin in 200 mL of methylene chloride (Example 15A). A second identical nifedipine-loaded organic solution was prepared. An aqueous solution composed of 1.8 g of ammonium bicarbonate in 20 mL of DI water was added to the first nifedipine organic solution (phase ratio 1:10). The mixture was homogenized for 5 minutes at 16,000 RPM. The nifedipine solution (Example 15A) and the nifedipine emulsion (Example 15B) were separately spray dried using process conditions of 20 mL/min solution flow rate, 60 kg/hr drying gas rate, and 21 °C outlet temperature.

### Example 16: Griseofulvin Drug Matrices Containing a Wetting Agent Produced With and Without a Pore Forming Agent

A griseofulvin/PEG solution was prepared by dissolving 5.0 g of griseofulvin, 11.2 g of PEG 3350, 11 mg of TWEEN™ 80, and 11 mg of lecithin in 200 mL of methylene chloride (Example 16A). A second identical griseofulvin-loaded organic solution was prepared. An aqueous solution composed of 1.8 g of ammonium bicarbonate in 20 mL of DI water was added to the first organic solution (phase ratio 1:10). The mixture was homogenized for 5 minutes at 16,000 RPM. The griseofulvin solution (Example 16A) and griseofulvin emulsion (Example 16B) were spray dried on a benchtop spray dryer using process conditions of 20 mL/min solution flow rate, 80 kg/hr drying gas rate, and 13 °C outlet temperature.

### Example 17: Internal Surface Area of Porous Drug Matrices Containing a Wetting Agent and Produced With and Without a Pore Forming Agent

The internal surface areas of the drug matrices produced in Examples 15 and 16 were assessed by Krypton BET. BET specific surface area analysis was performed using multi-point surface area analysis with krypton as the gas. Samples were outgassed to 20 micron vacuum at 20 °C prior to analysis. The results, shown in Table 4, illustrate that the use of the pore forming agent led to an increase of between 2.3 and 3.5 fold in the internal surface area of the resultant drug matrix.

**Table 4:**

| **Internal Surface Area of Drug Matrices** | |
|---|---|
| **Matrix** **(Example No.)** | **Surface Area** **(m²/g matrix)** |
| Nifedipine with wetting agent (15A) | 0.40 |
| Nifedipine with wetting agent and Ammonium Bicarbonate (15B) | 1.4 |
| Griseofulvin with wetting agent (16A) | 0.41 |
| Griseofulvin with wetting agent and Ammonium Bicarbonate (16B) | 0.95 |

### Example 18: Nifedipine Drug Matrix Produced Without a Pore Forming Agent or Wetting Agent

A 5% nifedipine solution was prepared by dissolving 10.0 g of nifedipine in 200 mL of methylene chloride. The solution was spray dried on a benchtop spray dryer using the following conditions: 20 mL/min solution flow rate, 60 kg/hr drying gas rate, and 22°C outlet temperature.

### Example 19: Griseofulvin Drug Matrix Produced Without a Pore Forming Agent or Wetting Agent

An 8.1% griseofulvin solution was prepared by dissolving 16.2 g of griseofulvin in 200 mL of methylene chloride. The solution was spray dried on a benchtop spray dryer using process conditions of 20 mL/min solution flow rate, 80 kg/hr drying gas rate, and 13 °C outlet temperature.

### Example 20: In Vitro Dissolution of Nifedipine Drug Matrices Produced With / Without Pore Forming Agent and Wetting Agent

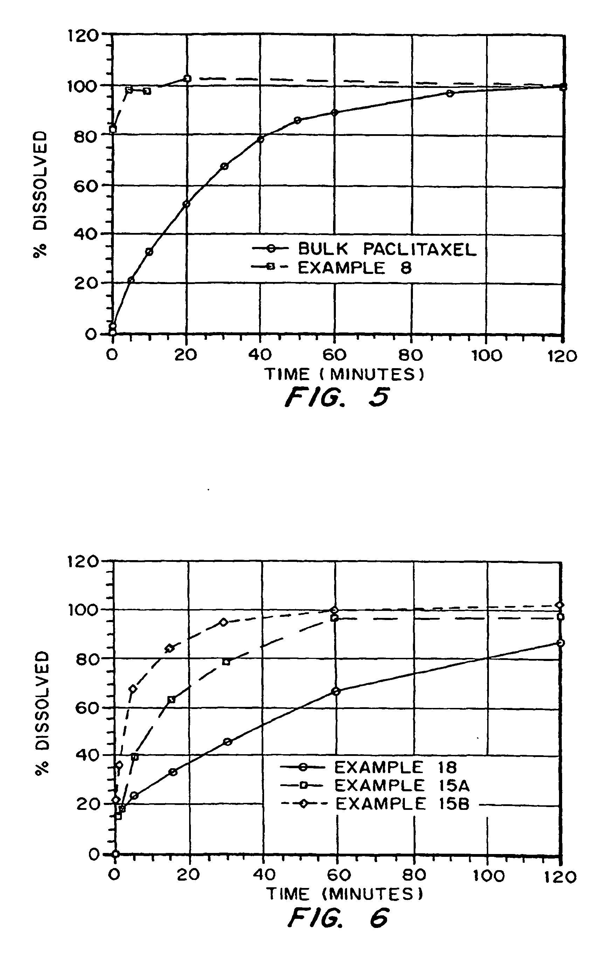

The *in vitro* dissolution rates of the nifedipine matrices produced in Examples 15 and 18 are shown in Figure 6. The *in vitro* dissolution of the drug matrices produced with either wetting agent or wetting agent and pore forming agent have increased dissolution rates as compared to the drug matrix produced with the drug alone. The matrix produced with both the wetting agent and the pore forming agent has the greatest dissolution rate.

### Example 21: In Vitro Dissolution of Griseofulvin Drug Matrices Produced With / Without Pore Forming Agent and Wetting Agent

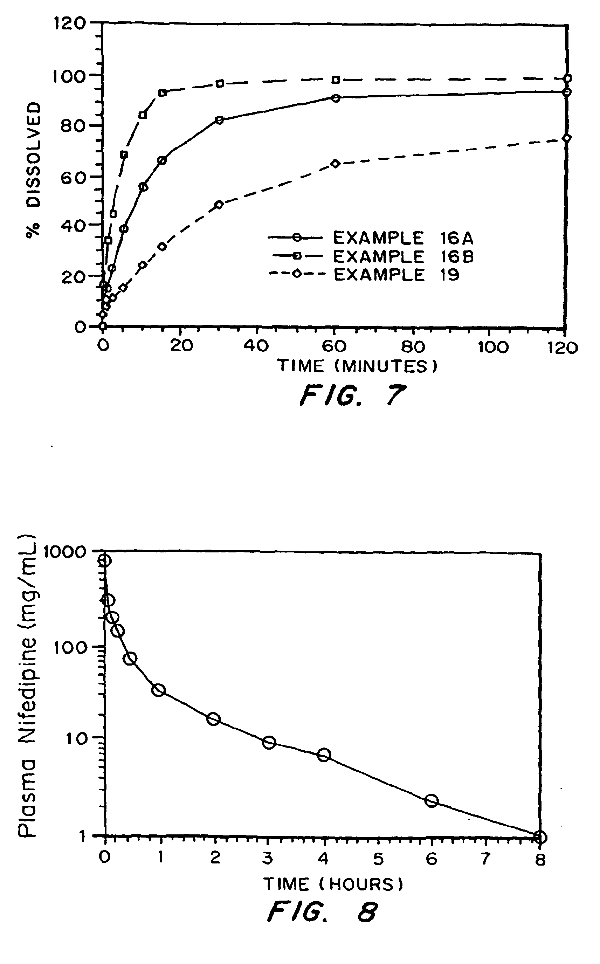

The *in vitro* dissolution rates of the griseofulvin matrices produced in examples 16 and 19 are provided in Figure 7. *The in vitro* dissolution of the drug matrices produced with either wetting agent or wetting agent and pore forming agent have increased dissolution rates as compared to the drug matrix produced with the drug alone. The matrix produced with both the wetting agent and the pore forming agent has the greatest dissolution rate.

### Example 22: Administration of Porous Drug Matrices as an Intravenous Bolus to Dogs

A nifedipine-loaded organic solution was prepared by dissolving 9.09 g of PEG 3350, 2.27 g of nifedipine, and 0.009 g of lecithin in 182 mL of methylene chloride. An aqueous solution was prepared by dissolving 3.27 g of ammonium bicarbonate and 0.91 g of PEG 3350 in 18.2 mL of deionized water at room temperature. The aqueous and organic solutions were homogenized as described in Example 1, and the resulting emulsion was spray dried using process conditions of 20 mL/min solution flow rate, 60 kg/hr drying gas rate, and 20 °C outlet temperature.

A suspension of the porous nifedipine drug matrix was prepared in 5% dextrose solution at a concentration of 2.5 mg/mL. The suspension (2 mL) was administered as a bolus to four beagle dogs, which weighed 8-10 kg. Blood samples were taken at time-points ranging from 1 minute to 24 hours. The samples were processed into plasma, were stored frozen, and were protected from light until analysis via liquid chromatography-mass spectrometry. All animals tolerated the suspension administered as a bolus. The average plasma levels of the intravenously administered suspension is shown in Figure 8.

### Example 23: Production of a Porous Nifedipine Matrix Using a Pegylated Phospholipid, 1, 2-Dimyristoyl-sn-Glycero-3-Phosphoethanolamine-N-[Poly(ethylene glycol)-5000]

A nifedipine-loaded organic solution was prepared by dissolving 2.0 g of nifedipine, 30.0 g of PEG 3350, 4 mg of lecithin, and 4 mg of 1, 2-Dimyristoyl-sn-Glycero-3-Phosphoethanolamine-N-[Poly(ethylene glycol)-5000] (PEG 5000 PE) in 202 mL of methylene chloride. An aqueous solution of 1.8 g of ammonium bicarbonate in 20 mL of DI water was added to the organic solution (phase ratio 1:10). The mixture was homogenized for 5 minutes at 16,000 RPM. The resulting emulsion was spray dried using process conditions of 20 mL/min solution flow rate, 60 kg/hr drying gas rate, and 21 °C outlet temperature.

### Example 24: Production of a Porous Nifedipine Matrix Using a Pegylated Phospholipid, 1, 2-Dimyristoyl-sn-Glycero-3-Phosphoethanolamine-N-[Poly(ethylene glycol)-2000]

A nifedipine-loaded organic solution was prepared by dissolving 2.0 g of nifedipine, 30.0 g of PEG 3350, 4 mg of lecithin, and 4 mg of 1, 2-Dimyristoyl-sn-Glycero-3-Phosphoethanolamine-N-[Poly(ethylene glycol)-2000] (PEG 2000 PE) in 202 mL of methylene chloride. An aqueous solution composed of 1.8 g of ammonium bicarbonate in 20 ml of DI water was added to the organic solution (phase ratio 1:10). The mixture was homogenized for 5 minutes at 16,000 RPM. The resulting emulsion was spray dried using process conditions of 20 mL/min solution flow rate, 60 kg/hr drying gas rate, and 21 °C outlet temperature.

Modifications and variations of the present invention will be obvious to those of skill in the art from the foregoing detailed description. Such modifications and variations are intended to come within the scope of the following claims.

## Claims

1. A method for making a porous matrix of drug comprising
(a) dissolving a drug in an organic volatile solvent to form a drug solution,
(b) combining at least one volatile solid pore forming agent with the drug solution to form an emulsion, suspension, or second solution containing the pore forming agent,
(c) incorporating at least one wetting agent into the emulsion, suspension or second solution, and
(d) removing the volatile solvent and pore forming agent from the emulsion, suspension, or second solution to yield the porous matrix of drug,
provided that the matrix is not formed by dissolving 18 grams of poly lactide-co-glycolide (50:50) (IV 0.4 dUg Boehringer Ingelheim), 1.08 g of diarachidoylphosphatidylcholine in 600 mL of methylene chloride to form a polymer solution; dissolving 38.9 mg of Eosin Y in 38.9 mL of a 0.18 g/ml ammonium bicarbonate solution to form an eosin solution; emulsifying the eosin solution with the polymer solution using a homogenizer at 7000 rpm for 8 minutes; pumping the solution at a flowrate of 20 mUmin and spray drying using a Lab spray dryer, wherein the inlet air temperature is 40°C, to form microparticles.

2. The method of claim 1 wherein the drug has low aqueous solubility.

3. The method of claim 1 wherein step (c) is conducted using a process selected from spray drying, evaporation, fluid bed drying, lyophilization, vacuum drying, or a combination thereof.

4. The method of claim 1 wherein the drug solution or pore forming agent further comprises an excipient selected from the group consisting of hydrophilic polymers, sugars, pegylated excipients, and tonicity agents.

5. The method of claim 1 wherein the pore forming agent is a volatile salt.

6. The method of claim 5 wherein the volatile salt is selected from the group consisting of ammonium bicarbonate, ammonium acetate, ammonium chloride, ammonium benzoate, and mixtures thereof.

7. The method of claim 1 which yields a porous matrix formed of a wetting agent and micorparticles of a drug, wherein the porous matrix upon contact with an aqueous medium yields microparticles of drug having a mean diameter between 0.1 and 5 µm and a total surface area greater than 0.5 m²/mL, and wherein the porous matrix is in a dry powder form.

8. A pharmaceutical composition comprising a porous matrix formed of a wetting agent and microparticles of a drug,
wherein the porous matrix upon contact with an aqueous medium yields microparticles of drug having a mean diameter between 0.1 and 5 µm and a total surface area greater than 0.5 m²/mL,
wherein the dry porous matrix is in a dry powder form, and wherein the porous matrix is made by a process comprising
(a) dissolving a drug in an organic volatile solvent to form a drug solution,
(b) combining at least one volatile solid pore forming agent with the drug solution to form an emulsion, suspension, or second solution,
(c) incorporating at least one wetting agent into the emulsion, suspension, or second solution, and
(d) removing the volatile solvent and pore forming agent from the emulsion, suspension, or second solution to yield the porous matrix,
provided that the matrix is not formed by dissolving 18 grams of poly lactide-co-glycolide (50:50) (IV 0.4 dL/g Boehringer Ingelheim), 1.08 g of diarachidoylphosphatidylcholine in 600 mL of methylene chloride to form a polymer solution; dissolving 38.9 mg of Eosin Y in 38.9 mL of a 0.18 g/ml ammonium bicarbonate solution to form an eosin solution; emulsifying the eosin solution with the polymer solution using a homogenizer at 7000 rpm for 8 minutes; pumping the solution at a flowrate of 20 mUmin and spray drying using a Lab spray dryer, wherein the inlet air temperature is 40°C, to form microparticles.

9. The composition of claim 8 wherein the porous matrix is in a dry powder form having a TAP density less than or equal to 1.0 g/mL.

10. The composition of claim 8 wherein the drug is a low aqueous solubility drug.

11. The composition of claim 10 wherein the matrix upon contact with an aqueous medium yields microparticles having a mean diameter between 0.1 and 5 µm and a total surface area greater than 0.5 m²/mL, and wherein the dry porous matrix is in a dry powder form having a TAP density less than or equal to 1.0 g/mL.

12. The composition of claim 8 wherein the drug is a taxane.

13. The composition of claim 12 wherein the taxane is paclitaxel or docetaxel.

14. The composition of claim 10 wherein the drug is selected from the group consisting of albuterol, adapalene, budesonide, doxazosin mesylate, mometasone furoate, ursodiol, amphotericin, enalapril maleate, felodipine, nefazodone hydrochloride; valrubicin, albendazole, estrogens conjugated, medroxyprogesterone acetate, nicardipine hydrochloride; zolpidem tartrate, amlodipine besylate, ethinyl estradiol, omeprazole, rubitecan, amlodipine besylate/ benazepril hydrochloride, etodolac, paroxetine hydrochloride, atovaquone, felodipine, podofilox, paricalcitol, betamethasone dipropionate, fentanyl, pramipexole dihydrochloride, Vitamin D₃ and related analogues, finasteride, quetiapine fumarate, alprostadil candesartan, cilexetil, fluconazole, ritonavir, busulfan, carbamazepine, flumazenil, risperidone, carbemazepine, carbidopa/levodopa, ganciclovir, saquinavir, amprenavir, carboplatin, glyburide, sertraline hydrochloride, rofecoxib carvedilol, halobetasolproprionate, sildenafil citrate, celecoxib, chlorthalidone, imiquimod, simvastatin, citalopram, ciprofloxacin, irinotecan hydrochloride, sparfloxacin, efavirenz, cisapride monohydrate, lansoprazole, tamsulosin hydrochloride, mofafinil, azithromycin, clarithromycin, letrozole, terbinafine hydrochloride, rosiglitazone maleate, diclofenac sodium, lomefloxacin hydrochloride, tirofiban hydrochloride, telmisartan, diazapam, loratadine, toremifene citrate, thalidomide, dinoprostone, mefloquine hydrochloride, trandolapril, mitoxantrone hydrochloride, tretinoin, etodolac, triamcinolone acetate, estradiol, ursodiol, nelfinavir mesylate, indinavir, beclomethasone dipropionate, oxaprozin, flutamide, famotidine, nifedipine, prednisone, cefuroxime, lorazepam, digoxin, lovastatin, griseofulvin, naproxen, ibuprofen, isotretinoin, tamoxifen citrate, nimodipine, amiodarone, and alprazolam.

15. The composition of claim 8 wherein the drug is water soluble.

16. The composition of claim 15 wherein the drug is selected from the group consisting of ceftriaxone, ketoconazole, ceftazidime, oxaprozin, albuterol sulfate, valacyclovic, urofollitropin, famciclovir, flutamide, cnalapril, metformin, itraconazole, buspirone, gabapentin, fosinopril, tramadol, acarbose, lorazepan, follitropin, glipizide, omeprazole, fluoxetine, lisinopril, levofloxacin, zafirlukast, interferon, growth hormone, interleukin, erythropoietin, granulocyte stiamlating factor, nizatidine, bupropion, perindopril, erbumine, adenosine, alendronate, alprostadil, benazepril, betaxolol, bleomycin sulfate, dexfenfluramine, diltiazem, fentanyl, flecainid, gemcitabine, glatiramer acetate, granisetron, lamivudine, mangafodipir trisodium, mesalamine, metoprolol fumarate, metronidazole, miglitol, moexipril, monteleukast, octreotide acetate, olopatadine, paricalcitol, somatropin, sumatriptan succinate, tacrince verapamil, nabumetone, trovafloxacin, dotasetron, zidovudine, finasteride, tobramycin, isradipine, toicapone, enoxaparin, fluconazole, lansopmzole, terbinafine, pamidronate, didanosine, diclofenac, cisapride, venlafaxine, troglitazone, fluvastatin, losartan, imiglucerase, donepezil, olanzapine, valsartan, fexofcnadine, calcitonin, and ipratropium.

17. The composition of claim 8 wherein the matrix further comprise an excipient selected from the group consisting of hydrophilic polymers, sugars, tonicity agents, pegylated excipients, and combinations thereof.

18. The composition of claim 8 wherein the mean diameter of the microparticles is between 1 and 5 µm.

19. The composition of claim 8 wherein the microparticles are suspended in an aqueous solution suitable for parenteral administration.

20. The composition of claim 8 wherein the matrix is processed into tablets or capsules suitable for oral administration.

21. The composition of claim 8 wherein the matrix is formed into suppositories suitable for vaginal or rectal administration.

22. The composition of claim 8 wherein the matrix is in a dry powder form suitable for pulmonary administration.

## Patentansprüche

1. Verfahren zur Herstellung einer porösen Matrix eines Wirkstoffs, umfassend:
(a) Lösen eines Wirkstoffs in einem organischen flüchtigen Lösungsmittel unter Bildung einer Wirkstofflösung,
(b) Kombinieren von zumindest einem flüchtigen festen porenbildenden Mittel mit der Wirkstofflösung unter Bildung einer Emulsion, Suspension oder einer zweiten Lösung enthaltend das porenbildende Mittel,
(c) Inkorporieren von zumindest einem Benetzungsmittel in die Emulsion, Suspension oder zweite Lösung, und
(d) Entfernen des flüchtigen Lösungsmittels und des porenbildenden Mittels aus der Emulsion, Suspension oder zweiten Lösung unter Erhalt der porösen Matrix des Wirkstoffs,
vorausgesetzt, daß die Matrix nicht gebildet wird durch Lösen von 18 g Polylactid-co-glycolid (50:50) (IV 0,4 dl/g Boehringer Ingelheim), 1,08 g Diarachidoylphosphatidylcholin in 600 ml Methylenchlorid unter Bildung einer Polymerlösung; Lösen von 38,9 mg Eosin Y in 38,9 ml einer 0,18 g/ml Ammoniumbicarbonatlösung unter Bildung einer Eosinlösung; Emulgieren der Eosinlösung mit der Polymerlösung unter Verwendung eines Homogenisators bei 7.000 U/min über 8 Minuten; Pumpen der Lösung bei einer Flußgeschwindigkeit von 20 ml/min und Sprühtrocknen unter Verwendung eines Laborsprühtrockners, worin die Einlaßlufttemperatur 40°C beträgt, unter Bildung von Mikropartikeln.

2. Verfahren nach Anspruch 1, worin der Wirkstoff eine niedere Löslichkeit in Wasser aufweist.

3. Verfahren nach Anspruch 1, worin Schritt (c) durchgeführt wird unter Verwendung eines Verfahrens ausgewählt aus Sprühtrocknen, Verdampfung, Fließbetttrocknung, Lyophilisierung, Vakuumtrocknen oder einer Kombination daraus.

4. Verfahren nach Anspruch 1, worin die Wirkstofflösung oder das porenbildende Mittel weiterhin einen Trägerstoff ausgewählt aus der Gruppe bestehend aus hydrophilen Polymeren, Zuckern, PEGylierten Trägerstoffen und Tonizitätsmitteln umfaßt.

5. Verfahren nach Anspruch 1, worin das porenbildende Mittel eine flüchtiges Salz ist.

6. Verfahren nach Anspruch 5, worin das flüchtige Salz ausgewählt ist aus der Gruppe bestehend aus Ammoniumbicarbonat, Ammoniumacetat, Ammoniumchlorid, Ammoniumbenzoat und Mischungen daraus.

7. Verfahren nach Anspruch 1, welches eine aus einem Benetzungsmittel und Mikropartikeln eines Wirkstoffs gebildete poröse Matrix ergibt, worin die poröse Matrix bei Kontakt mit einem wäßrigen Medium Mikropartikel des Wirkstoffs mit einem mittleren Durchmesser zwischen 0,1 und 5 µm und einer Gesamtoberfläche von mehr als 0,5 m²/ml ergibt, und worin die poröse Matrix in einer trockenen Pulverform vorliegt.

8. Pharmazeutische Zusammensetzung umfassend eine poröse Matrix gebildet aus einem Benetzungsmittel und Mikropartikeln eines Wirkstoffs,
worin die poröse Matrix bei Kontakt mit einem wäßrigen Medium Mikropartikel eines Wirkstoffs mit einem mittleren Durchmesser zwischen 0,1 und 5 µm und einer Gesamtoberfläche von mehr als 0,5 m²/ml ergibt,
worin die trockene poröse Matrix in trockener Pulverform vorliegt, und worin die poröse Matrix gebildet wird durch ein Verfahren umfassend:
(a) Lösen eines Wirkstoffs in einem organischen flüchtigen Lösungsmittel unter Bildung einer Wirkstofflösung,
(b) Kombinieren von zumindest einem festen flüchtigen porenbildenden Mittel mit der Wirkstofflösung unter Bildung einer Emulsion, Suspension oder zweiten Lösung,
(c) Inkorporieren von zumindest einem Benetzungsmittel in die Emulsion, Suspension oder zweite Lösung, und
(d) Entfernen des flüchtigen Lösungsmittels und porenbildenden Mittels aus der Emulsion, Suspension oder zweiten Lösung unter Erhalt der porösen Matrix,
vorausgesetzt, daß die Matrix nicht gebildet wird durch Lösen von 18 g Polylactid-co-glycolid (50:50) (IV 0,4 dl/g Boehringer Ingelheim), 1,08 g Diarachidoylphosphatidylcholin in 600 ml Methylenchlorid unter Bildung einer Polymerlösung; Lösen von 38,9 mg Eosin Y in 38,9 ml einer 0,18 g/ml Ammoniumbicarbonatlösung unter Bildung einer Eosinlösung; Emulgieren der Eosinlösung mit der Polymerlösung unter Verwendung eines Homogenisators bei 7.000 U/min über 8 Minuten; Pumpen der Lösung bei einer Flußgeschwindigkeit von 20 ml/min und Sprühtrocknen unter Verwendung eines Laborsprühtrockners, worin die Einlaßlufttemperatur 40°C beträgt, unter Bildung von Mikropartikeln.

9. Zusammensetzung nach Anspruch 8, worin die poröse Matrix in einer trockenen Pulverform vorliegt mit einer TAP-Dichte von weniger oder gleich 1,0 g/ml.

10. Zusammensetzung nach Anspruch 8, worin der Wirkstoff ein Wirkstoff mit geringer wäßriger Löslichkeit ist.

11. Zusammensetzung nach Anspruch 10, worin die Matrix bei Kontakt mit einem wäßrigen Medium Mikropartikel mit einem mittleren Durchmesser zwischen 0,1 und 5 µm und einer Gesamtoberfläche von mehr als 0,5 m²/ml ergibt, und worin die trockene poröse Matrix in einer trockenen Pulverform mit einer TAP-Dichte von weniger oder gleich 1,0 g/ml vorliegt.

12. Zusammensetzung nach Anspruch 8, worin der Wirkstoff ein Taxan ist.

13. Zusammensetzung nach Anspruch 12, worin das Taxan Paclitaxel oder Docetaxel ist.

14. Zusammensetzung nach Anspruch 10, worin der Wirkstoff ausgewählt ist aus der Gruppe bestehend aus Albuterol, Adapalen, Budesonid, Doxazosinmesylat, Mometasonfuroat, Ursodiol, Amphotericin, Enalaprilmaleat, Felodipin, Nefazodonhydrochlorid, Valrubicin, Albendazol, konjugierte Östrogene, Medroxyprogesteronacetat, Nicardipinhydrochlorid, Zoldipemtartrat, Amlodipinbesylat, Ethinylestradiol, Omeprazol, Rubitecan, Amlodipinbesylat/Benazeprilhydrochlorid, Etodolac, Paroxetinhydrochlorid, Atovaquon, Felodipin, Podofilox, Paricalcitol, Betamethasondipropionat, Fentanyl, Pramipexoldihydrochlorid, Vitamin D3 und verwandte Analoga, Finasterid, Quetiapinfumarat, Alprostadil, Candesartan, Cilexetil, Fluconazol, Ritonavir, Busulfan, Carbamazepin, Flumazenil, Risperidon, Carbemazepin, Carbidopa/Levodopa, Ganciclovir, Saquinavir, Amprenavir, Carboplatin, Glyburid, Sertralinhydrochlorid, Rofecoxib, Carvedilol, Halobetasolproprionat, Sildenafilcitrat, Celecoxib, Chlorthalidon, Imiquimod, Simvastatin, Citalopram, Ciprofloxacin, Irinotecanhydrochlorid, Sparfloxacin, Efavirenz, Cisapridmonohydrat, Lansoprazol, Tamsulosinhydrochlorid, Modafinil, Azithromycin, Clarithromycin, Letrozol, Terbinafinhydrochlorid, Rosiglitazonmaleat, Diclofenacnatrium, Lomefloxacinhydrochlorid, Tirofibanhydrochlorid, Telmisartan, Diazepam, Loratadin, Toremifencitrat, Thalidomid, Dinoproston, Mefloquinhydrochlorid, Trandolapril, Mitoxantronhydrochlorid, Tretinoin, Etodolac, Triamcinolonacetat, Estradiol, Ursodiol, Nelfinavirmesylat, Indinavir, Beclomethasondipropionat, Oxaprozin, Flutamid, Famotidin, Nifedipin, Prednison, Cefuroxim, Lorazepam, Digoxin, Lovastatin, Griseofulvin, Naproxen, Ibuprofen, Isotretinoin, Tamoxifencitrat, Nimodipin, Amiodaron und Alprazolam.

15. Zusammensetzung nach Anspruch 8, worin der Wirkstoff wasserlöslich ist.

16. Zusammensetzung nach Anspruch 15, worin der Wirkstoff ausgewählt ist aus der Gruppe bestehend aus Ceftriaxon, Ketoconazol, Ceftazidim, Oxaprozin, Albuterolsulfat, Valacyclovir, Urofollitropin, Famciclovir, Flutamid, Enalapril, Metformin, Itraconazol, Buspiron, Gabapentin, Fosinopril, Tramadol, Acarbose, Lorazepan, Follitropin, Glipizid, Omeprazol, Fluoxetin, Lisinopril, Levofloxacin, Zafirlukast, Interferon, Wachstumshormon, Interleukin, Erythropoietin, Granulocyten-stimulierendem Faktor, Nizatidin, Bupropion, Perindopril, Erbumin, Adenosin, Alendronat, Alprostadil, Benazepril, Betaxolol, Bleomycinsulfat, Dexfenfluramin, Diltiazem, Fentanyl, Flecainid, Gemcitabin, Glatirameracetat, Granisetron, Lamivudin, Mangafodipirtrinatrium, Mesalamin, Metoprololfumarat, Metronidazol, Miglitol, Moexipril, Montelukast, Octreotidacetat, Olopatadin, Paricalcitol, Somatropin, Sumatriptansuccinat, Tacrin, Verapamil, Nabumeton, Trovafloxacin, Dolasetron, Zidovudin, Finasterid, Tobramycin, Isradipin, Tolcapon, Enoxaparin, Fluconazol, Lansoprazol, Terbinafin, Pamidronat, Didanosin, Diclofenac, Cisaprid, Venlafaxin, Troglitazon, Fluvastatin, Losartan, Imiglucerase, Donepezil, Olanzapin, Valsartan, Fexofenadin, Calcitonin und Ipratropium.

17. Zusammensetzung nach Anspruch 8, worin die Matrix weiterhin einen Trägerstoff ausgewählt aus der Gruppe bestehend aus hydrophilen Polymeren, Zuckern, Tonizitätsmitteln, PEGylierten Trägerstoffen und Kombinationen daraus umfaßt.

18. Zusammensetzung nach Anspruch 8, worin der mittlere Durchmesser der Mikropartikel zwischen 1 und 5 µm liegt.

19. Zusammensetzung nach Anspruch 8, worin die Mikropartikel in einer zur parenteralen Verabreichung geeigneten wäßrigen Lösung suspendiert sind.

20. Zusammensetzung nach Anspruch 8, worin die Matrix in zur oralen Verabreichung geeigneten Kapseln oder Tabletten prozessiert ist.

21. Zusammensetzung nach Anspruch 8, worin die Matrix in zur rektalen oder vaginalen Verabreichung geeigneten Suppositorien gebildet ist.

22. Zusammensetzung nach Anspruch 8, worin die Matrix in einer zur pulmonaren Verabreichung geeigneten trockenen Pulverform vorliegt.

## Revendications

1. Procédé de fabrication d'une matrice poreuse de médicament comprenant
(a) la dissolution d'un médicament dans un solvant organique volatil pour former une solution médicamenteuse,
(b) la combinaison d'au moins un agent porogène solide volatil avec la solution médicamenteuse pour former une émulsion, une suspension ou une seconde solution contenant l'agent porogène,
(c) l'incorporation d'au moins un agent mouillant dans l'émulsion, la suspension ou la seconde solution, et
(d) le retrait du solvant volatil et de l'agent porogène de l'émulsion, de la suspension ou de la seconde solution pour produire la matrice poreuse de médicament,
à condition que la matrice ne soit pas formée en dissolvant 18 grammes de poly(lactide-co-glycolide) (50:50) (IV 0,4 dl/g Boehringer Ingelheim), 1,08 g de diarachidoylphosphatidylcholine dans 600 ml de chlorure de méthylène pour former une solution de polymère ; en dissolvant 38,9 mg d'éosine Y dans 38,9 ml d'une solution de bicarbonate d'ammonium à 0,18 g/ml pour former une solution d'éosine ; en émulsionnant la solution d'éosine avec la solution de polymère à l'aide d'un homogénéisateur à 7000 tr/mn pendant 8 minutes ; en pompant la solution à un débit de 20 ml/mn et en la séchant par atomisation à l'aide d'un sécheur-atomiseur Lab, dans lequel la température de l'air à l'entrée est de 40°C, pour former des microparticules.

2. Procédé selon la revendication 1 dans lequel le médicament possède une faible solubilité aqueuse.

3. Procédé selon la revendication 1 dans lequel l'étape (c) est effectuée en utilisant un procédé choisi parmi le séchage par atomisation, l'évaporation, le séchage en lit fluidisé, la lyophilisation, le séchage sous vide, ou une combinaison de ceux-ci.

4. Procédé selon la revendication 1 dans lequel la solution médicamenteuse, ou l'agent porogène comprend en outre un excipient choisi dans le groupe constitué par les polymères hydrophiles, les sucres, les excipients pégylés et les agents de tonicité.

5. Procédé selon la revendication 1 dans lequel l'agent porogène est un sel volatil.

6. Procédé selon la revendication 5 dans lequel le sel volatil est choisi dans le groupe constitué par le bicarbonate d'ammonium, l'acétate d'ammonium, le chlorure d'ammonium, le benzoate d'ammonium et les mélanges de ceux-ci.

7. Procédé selon la revendication 1 qui produit une matrice poreuse formée d'un agent mouillant et de microparticules d'un médicament, dans lequel la matrice poreuse, au contact d'un milieu aqueux, produit des microparticules de médicament possédant un diamètre moyen entre 0,1 et 5 µm et une surface spécifique totale supérieure à 0,5 m²/ml, et dans lequel la matrice poreuse est sous la forme d'une poudre sèche.

8. Composition pharmaceutique comprenant une matrice poreuse formée d'un agent mouillant et de microparticules d'un médicament,
dans laquelle la matrice poreuse, au contact d'un milieu aqueux, produit des microparticules de médicament possédant un diamètre moyen entre 0,1 et 5 µm et une surface spécifique totale supérieure à 0,5 m²/ml,
dans laquelle la matrice poreuse sèche est sous la forme d'une poudre sèche, et
dans laquelle la matrice poreuse est fabriquée par un procédé comprenant
(a) la dissolution d'un médicament dans un solvant organique volatil pour former une solution médicamenteuse,
(b) la combinaison d'au moins un agent porogène solide volatile avec la solution médicamenteuse pour former une émulsion, une suspension ou une seconde solution,
(c) l'incorporation d'au moins un agent mouillant dans l'émulsion, la suspension ou la seconde solution, et
(d) le retrait du solvant volatil et de l'agent porogène de l'émulsion, de la suspension ou de la seconde solution pour produire la matrice poreuse,
à condition que la matrice ne soit pas formée en dissolvant 18 grammes de poly(lactide-co-glycolide) (50:50) (IV 0,4 dl/g Boehringer Ingelheim), 1,08 g de diarachidoylphosphatidylcholine dans 600 ml de chlorure, de méthylène pour former une solution de polymère ; en dissolvant 38,9 mg d'éosine Y dans 38,9 ml d'une solution de bicarbonate d'ammonium à 0,18 g/ml pour former une solution d'éosine ; en émulsionnant la solution d'éosine avec la solution de polymère à l'aide d'un homogénéisateur à 7000 tr/mr. pendant 8 minutes ; en pompant la solution à un débit des 20 ml/mn et en la séchant par atomisation à l'aide d'un sécheur-atomiseur Lab, dans lequel la température de l'air à l'entrée est de 40°C, pour former des microparticules.

9. Composition selon la revendication 8 dans laquelle la matrice poreuse est sous la forme d'une poudre sèche possédant une masse volumique après tassement inférieure ou égale à 1,0 g/ml.

10. Composition selon la revendication 8 dans laquelle le médicament est un médicament à faible solubilité aqueuse.

11. Composition selon la revendication 10 dans laquelle la matrice, au contact d'un milieu aqueux, produit des microparticules possédant un diamètre moyen entre 0,1 et 5 µm et une surface spécifique totale supérieure à 0,5 m²/ml, et dans laquelle la matrice poreuse sèche est sous la forme d'une poudre sèche possédant une masse volumique après tassement inférieure ou égale à 1,0 g/ml.

12. Composition selon la revendication 8 dans laquelle le médicament est un taxane.

13. Composition selon la revendication 12 dans laquelle le taxane est le paclitaxel ou le docétaxel.

14. Composition selon la revendication 10 dans laquelle le médicament est choisi dans le groupe constitué par l'albutérol, l'adapalène, le budésonide, le mésylate de doxazosine, le furoate de mométasone, l'ursodiol, l'amphotéricine, le maléate d'énalapril, la félodipine, le chlorhydrate de néfazodone, la valrubicine, l'albendazole, les oestrogènes conjugués, l'acétate de médroxyprogestérone, le chlorhydrate de nicardipine, le tartrate de zolpidem, le bésylate d'amlodipine, l'éthinyloestradiol, l'oméprazole, le rubitécan, le bésylate d'amlodipine/chlorhydrate de bénazépril, l'étodolac, le chlorhydrate de paroxétine, l'atovaquone, la félodipine, le podofilox, le paricalcitol, le dipropionate de bétaméthasone, le fentanyl, le dichlorhydrate de pramipexole, la vitamine D₃ et les analogues apparentés, le finastéride, le fumarate de quétiapine, l'alprostadil, le candésartan, le cilexétil, le fluconazole, le ritonavir, le busulfan, la carbamazépine, le flumazénil, la rispéridone, la carbémazépine, le carbidopa/lévodopa, le ganciclovir, le saquinavir, l'amprénavir, le carboplatine, le glyburide, le chlorhydrate de sertraline, le rofécoxib, le carvédiol, le propionate d'halobétasol, le citrate de sildénafil, le célécoxib, la chlorthalidone, l'imiquimod, la simvastatine, le citalopram, la ciprofloxacine, le chlorhydrate d'irinotécan, la sparfloxacine, l'éfavirenz, le monohydrate de cisapride, le lansoprazole, le chlorhydrate de tamsulosine, le modafinil, l'azithromycine, la clarithromycine, le létrozole, le chlorhydrate de terbinafine, le maléate de rosiglitazone, le diclofénac sodique, le chlorhydrate de loméfloxacine, le chlorhydrate de tirofiban, le telmisartan, le diazépam, la loratadine, le citrate de torémifène, le thalidomide, la dinoprostone, le chlorhydrate de méfloquine, le trandolapril, le chlorhydrate de mitoxantrone, la trétinoëne, l'étodolac, l'acétate de triamcinolone, l'oestradiol, l'ursodiol, le mésylate de nelfinavir, l'indinavir, le dipropionate de béclométhasone, l'oxaprozine, le flutamide, la famotidine, la nifédipine, la prednisone, le céfuroxime, le lorazépam, la digoxine, la lovastatine, la griséofulvine, le naproxène, l'ibuprofène, l'isotrétinoïne, le citrate de tamoxifène, la nimodipine, l'amiodarone et l'alprazolam.

15. Composition selon la revendication 8 dans laquelle le médicament est hydrosoluble.

16. Composition selon la revendication 15 dans laquelle le médicament est choisi dans le groupe constitué par la ceftriaxone, le kétoconazole, la ceftazidime, l'oxaprozine, le sulfate d'albutérol, le valacyclovir, l'urofollitropine, le famciclovir, le flutamide, l'énalapril, la metformine, l'itraconazole, la buspirone, la gabapentine, le fosinopril, le tramadol, l'acarbose, le lorazépam, la follitropine, le glipizide, l'oméprazole, la fluoxétine, le lisinopril, la lévofloxacine, le zafirlukast, l'interféron, l'hormone de croissance, l'interleukine, l'érythropoïétine, le facteur de stimulation des granulocytes, la nizatidine, le bupropion, le perindopril, l'erbumine, l'adénosine, l'alendronate, l'alprostadil, le bénazépril, le bétaxolol, le sulfate de bléomycine, la dexfenfluramine, le diltiazem, le fentanyl, le flécaïnide, la gemcitabine, l'acétate de glatiramer, le granisétron, la lamivudine, le mangafodipir trisodique, la mésalamine, le fumarate de métoprolol, le métronidazole, le miglitol, le moexipril, le montélukast, l'acétate d'octréotide, l'olopatadine, le paricalcitol, la somatropine, le succinate de sumatriptan, la tacrine, le vérapamil, la nabumétone, la trovafloxacine, le dolasétron, la zidovudine, le finastéride, la tobramycine, l'isradipine, la tolcapone, l'énoxaparine, le fluconazole, le lansoprazole, la terbinafine, le pamidronate, la didanosine, le diclofénac, le cisapride, la venlafaxine, la troglitazone, la fluvastatine, le losartan, l'imiglucérase, le donépézil, l'olanzapine, le valsartan, la fexofénadine, la calcitonine et l'ipratropium.

17. Composition selon la revendication 8 dans laquelle la matrice comprend en outre un excipient choisi dans le groupe constitué par les polymères hydrophiles, les sucres, les agents de tonicité, les excipients pégylés et les combinaisons de ceux-ci.

18. Composition selon la revendication 8 dans laquelle le diamètre moyen des microparticules est entre 1 et 5 µm.

19. Composition selon la revendication 8 dans laquelle les microparticules sont en suspension dans une solution aqueuse appropriée pour administration parentérale.

20. Composition selon la revendication 8 dans laquelle la matrice est transformée en comprimés ou en capsules appropriés pour administration orale.

21. Composition selon la revendication 8 dans laquelle la matrice est façonnée en suppositoires appropriés pour administration vaginale ou rectale.

22. Composition selon la revendication 8 dans laquelle la matrice est sous la forme d'une poudre sèche appropriée pour administration pulmonaire.
